# EUROPEAN PATENT APPLICATION

(11) **EP 1 347 051 A1**
(43) Date of publication of application: **24.09.2003**
(21) Application number: 01272292.2
(22) Date of filing: 21.12.2001
(51) Int. Cl.: C12N 15/12, C12Q 1/68, C12Q 1/02, A01K 67/027, A61K 31/713, A61K 45/00, A61K 48/00, A61P 37/08, G01N 33/15, G01N 33/50, G01N 33/53

(54) **METHOD OF EXAMINING ALLERGIC DISEASE**

(30) Priority: 26.12.2000 JP 2000396166
(71) Applicant: Genox Research, Inc., Tsukuba-shi Ibaraki 300-2635 (JP)
(72) Inventor: OHTANI, N. Genox Research, Inc., Kawasaki-shi, Kanagawa 216-0001 (JP); MATSUI, K. Genox Research, Inc., Kawasaki-shi, Kanagawa 216-0001 (JP); YOSHIDA, N. Genox Research, Inc., Kawasaki-shi, Kanagawa 216-0001 (JP); SUGITA, Y. Genox Research, Inc., Kawasaki-shi, Kanagawa 216-0001 (JP); IZUHARA, K. Department of Biochemistry, Saga-shi, Saga 849-8501 (JP)
(74) Representative: Warcoin, Jacques
(86) International application number: JP0111287
(87) International publication number: WO02052006

(57) **Abstract**

Six genes, whose expressions were greatly changed in a plurality of cells by stimulating respiratory tract epithelial cells with IL-4 or IL-13, were obtained as allergy related genes. This invention provides a method of testing for allergic diseases, and method of screening for compounds useful in treating such diseases, that use as indicators, expression levels of these genes in biological samples.

## Description

### Technical Field

The present invention relates to a method of testing for an allergic disease.

### Background Art

Bronchial asthma is considered to be a multifactorial disease. In other words, bronchial asthma is caused by the interaction of many different genes, each of which is influenced by various environmental factors. Thus, it has been extremely difficult to identify a specific gene which causes bronchial asthma.

Currently, bronchial asthma is categorized as a chronic inflammatory disease of the respiratory tract. It has been pointed out that allergic reactions at the respiratory tract mucosa and bronchial smooth muscle is closely involved in pathologic formation of bronchial asthma. Therefore, understanding the condition of allergic reactions in these tissues is an important issue in diagnosis of bronchial asthma. In addition, control of allergic reactions is an issue in treatment of bronchial asthma.

On the other hand, the expression of mutated or defective genes, or overexpression or reduction of the expression of specific gene is thought to be involved in allergic diseases. To elucidate the role of gene expression in diseases, it is necessary to understand how a gene is involved in triggering disease onset and how expression of the gene is altered by external stimulants such as drugs.

Incidentally, atopic diathesis that is accompanied by hyperproduction of IgE antibodies is seen in many bronchial asthma patients. Many causes are considered for bronchial asthma, but there is no doubt that atopic diathesis is a cause of hypersensitivity in many patients. It has been predicted that the mechanism of respiratory tract occlusion in asthmatic attack is contraction of the bronchial smooth muscle, or edema and respiratory tract endocrine enhancement of the respiratory tract mucosa. I-type allergic reaction in the respiratory tract due to exposure to pathogenic allergen has an important role in such changes in the respiratory tract.

In recent years, IL-4 and IL-13 have been suggested to have important roles in the onset of bronchial asthma. Therefore, for example, in the respiratory tract epithelial cells and bronchial smooth muscles, genes that change their expression level due to IL-4 and IL-13 are thought to be related to bronchial asthma. However, based on such concept, there have been no reports on isolation of genes that specifically change their expression level due to IL-4 and IL-13.

In recent diagnosis of allergic diseases, history taking, and confirmation of the patient's family history and own anamnesis are important factors in general. In addition, for diagnosis of allergy based on more objective information, a test method using patient's blood sample and method for observing patient's immune response to allergen are also performed. Examples of the former method are the allergen-specific IgE measurement, leukocyte histamine release test, lymphocyte stimulating test, or the like. Presence of an allergen-specific IgE is a proof for the allergic reaction to that allergen. However, in some patients, allergen-specific IgE may not necessarily be detected. Furthermore, the assay principle of IgE requires performing tests for all of the allergens necessary for diagnosis. Leukocyte histamine release test and lymphocyte stimulating test are the methods for observing the immune system reaction toward a specific allergen *in vitro.* These methods are complicate in operation.

On the other hand, another method is also known, wherein the immune response observed when a patient is actually contacted with an allergen is used for diagnosing an allergy (latter method). Such a test includes the prick test, scratch test, patch test, intradermal reaction, or induction test. Indeed these tests allow the direct diagnosis of patient's allergic reaction but they can be said to be highly invasive tests wherein patients are actually exposed to allergen.

In addition, regardless of the allergen types, test methods for proving the involvement of allergic reaction are also attempted. For example, a high serum IgE titer may indicate the occurrence of allergic reaction in the patient. The serum IgE titer is information corresponding to the total amount of allergen-specific IgE. Though it is easy to determine the total amount of IgE regardless of the type of allergen, IgE titer may be reduced in some patients with a non-atopic bronchitis or the like.

Therefore, a marker (indicator) for an allergic disease that is not only less risky to patients but also capable of readily providing information necessary for diagnosis would be useful. Since such markers are thought to be profoundly involved in triggering disease onset, they may become the important target in not only diagnosis but also control of allergic symptoms.

### Disclosure of the Invention

An objective of the present invention is to provide an indicator enabling the test for allergic disease, in particular. Another objective of the invention is to provide a method of testing for an allergic disease and a method of screening for a candidate compound for a therapeutic agent for an allergic disease based on the indicator.

Deep involvement of IL-4 and IL-13 in allergic reaction has been suggested by several reports. For example, in an IL-4 knockout mouse (Yssel, H and Groux, H: Int. Arch. Allergy Immunol., 121; 10-18, 2000) and in a STAT6 knockout mouse (Akimoto, T. et al.: J. Exp. Med., 187, 1537-1542, 1998), respiratory tract hypersensitivity disappears. In a mouse model, IL-13 is involved in forming asthma-like pathology regardless of IgE production and Th2 type (Wills-Karp, M. et al.: Science, 282, 2258-2261, 1998; Grunig, G. et al.: Science, 282, 2261-2263, 1998; Zhu, Z. etal. : J. Clin. Invest., 103, 779-788, 1999).

Additionally, IL-4 receptors and IL-13 receptors are highly expressed in human respiratory tract epithelial cells and bronchial smooth muscles (Heinzmann, A. et al.; Hum. Mol. Genet., 9: 549-559, 2000). Accordingly, these tissues are thought to be target cells of 11-4 and IL-13. On the other hand, SNP present in IL-4 receptor α and IL-13 were shown to be one of the genetic causes of allergic diseases (Mitsuyasu, H., et al.: Nature Genet., 19, 119-120, 1998; Mitsuyasu, H., et al.: J. Immunol., 162: 1227-1231, 1999; Kruse, S., et al.: Immnol., 96, 365-371, 1999; Heinzmann, A. et al.: Hum. Mol. Genet., 9: 549-559, 2000). Furthermore, inhibition of IL-4 or IL-13 function by soluble IL-4 receptor α was shown to be effective as treatment for bronchial asthma (Borish, L. C. et al.: Am. J. Respir. Crit. Care Med., 160: 912-922, 1999).

According to the above, a strong relationship to allergic reactions, mainly respiratory symptoms in particular, has been suggested for IL-4 and IL-13. That is, genes constituting signal transduction pathway due to IL-4 and IL-13 may be genes that are closely related to allergic reactions. Therefore, by isolating these genes and elucidating their relation to allergic reactions, novel targets for treatment of allergic diseases can be found.

Based on such line of thought, the inventors thought that, if genes indicating changes in expression levels were searched for when human bronchial epithelial cells were treated with IL-4 and IL-13, genes relating to allergic reactions could be isolated. There is a report that attempted, using a similar approach, to isolate genes whose expression level changed by IL-4 and IL-13 treatment (Wang et al., Immunology 2000, Seattle, May 12-16, 2000). However, since in known searching methods, the number of lots of cells used for analysis is small, and the range of changes in expression levels are not clear, specificity towards stimulation from IL-4 and IL-13 cannot be expected.

Therefore, to isolate genes that respond with higher specificity towards IL-4 and IL-13 stimulation, the inventors increased the number of lots of cells that become the object of analysis, and furthermore, selected those in which the change in expression level reached twice as much or more. Next, in the respiratory tract epithelial cells stimulated with IL-4 and IL-13, significant elevation of expression level of genes selected this way was confirmed. Based on the findings mentioned above, the inventors succeeded in elucidating the existence of the following 6 genes, which have close relationship to allergic diseases.
carboxypeptidase M
cathepsin C
endothelin-A receptor
osteoblast specific factor 2 (OSF2os)
DD96(MAP17)
and dioxin-inducible cytochrome P450 (CYP1B1)

Based on the findings mentioned above, the inventors found that tests for allergic diseases become possible by using these genes and proteins encoded by these genes as indicators, and completed this invention. Additionally, the inventors found that screening of therapeutic agents for allergic diseases is possible by using expression levels of these genes or activities of proteins encoded by these genes as indicators, and completed this invention.

That is, this invention relates to the following testing method and screening methods.
[1] A method of testing for an allergic disease, the method comprising the steps of:
   a) measuring the expression level of one or more genes selected from the group consisting of carboxypeptidase M, cathepsin C, endothelin-A receptor, osteoblast specific factor 2, DD96, and CYP1B1 in a biological sample from a subject, and
   b) comparing the expression level measured in (a) with that in a biological sample from a normal healthy subject.
[2] The method of [1], wherein the allergic disease is bronchial asthma.
[3] The method of [1], wherein the expression level is measured by PCR of the cDNA for the one or more genes.
[4] The method of [1] wherein the expression level is measured by detecting the protein encoded by the one or more genes.
[5] A reagent for testing for an allergic disease, said reagent comprising an oligonucleotide that is at least 15 nucleotides long and that has a nucleotide sequence complementary to a polynucleotide having a nucleotide sequence of one or more genes selected from the group consisting of carboxypeptidase M, cathepsin C, endothelin-A receptor, osteoblast specific factor 2, DD96(MAP17), and CYP1B1 or to a complementary strand of the polynucleotide.
[6] A reagent for testing for an allergic disease, said reagent comprising an antibody that recognizes a peptide having an amino acid sequence of one or more proteins selected from the group consisting of carboxypeptidase M, cathepsin C, endothelin-A receptor, osteoblast specific factor 2, DD96 (MAP17), and CYP1B1.
[7] A method of screening for a therapeutic agent for an allergic disease, the method comprising the steps of:
   (1) contacting a candidate compound with a cell that expresses one or more genes selected from the group consisting of carboxypeptidase M, cathepsin C, endothelin-A receptor, osteoblast specific factor 2, DD96(MAP17), and CYP1B1, and/or one or more genes functionally equivalent thereto,
   (2) measuring the expression level of the one or more genes, and
   (3) selecting a compound that lowers the expression level, compared to a control.
[8] The method of [7], wherein the cell is a respiratory tract epithelial cell line.
[9] A method of screening for a therapeutic agent for an allergic disease, the method comprising the steps of:
   (1) administering a candidate compound to a test animal,
   (2) measuring, in a biological sample from the test animal, the expression level of one or more genes selected from the group consisting of carboxypeptidase M, cathepsin C, endothelin-A receptor, osteoblast specific factor 2, DD96(MAP17), and CYP1B1, and/or one or more genes functionally equivalent thereto, and
   (3) selecting a compound that lowers the expression level of the one or more genes, compared to a control.
[10] A method of screening for a therapeutic agent for an allergic disease, the method comprising the steps of:
   (1) contacting a candidate substance with a cell transfected with a vector having a transcription regulatory region of one or more genes selected from the group consisting of carboxypeptidase M, cathepsin C, endothelin-A receptor, osteoblast specific factor 2, DD96(MAP17), and CYP1B1, and/or one or more genes functionally equivalent thereto, and a reporter gene that is expressed under the control of the transcription regulatory region,
   (2) measuring activity of the reporter gene, and
   (3) selecting a compound that lowers the expression level of the reporter gene, compared to a control.
[11] A method of screening for a therapeutic agent for an allergic disease, the method comprising the steps of:
   (1) contacting a candidate substance with one or more proteins selected from the group consisting of carboxypeptidase M, cathepsin C, endothelin-A receptor, osteoblast specific factor 2, DD96 (MAF17) and CYP1B1, and/or one or more proteins functionally equivalent thereto,
   (2) measuring activity of the one or more proteins, and
   (3) selecting a compound that lowers the activity, compared to a control.
[12] A therapeutic agent for an allergic disease, said agent comprising, as an active ingredient, a compound obtained by the method of any one of [7] , [9] , [10] , and [11].
[13] A therapeutic agent for an allergic disease, said agent comprising, as a major component, an antisense DNA against one or more genes selected from the group consisting of carboxypeptidase M, cathepsin C, endothelin-A receptor, osteoblast specific factor 2, DD96(MAP17), and CYP1B1 or against a portion thereof.
[14] A therapeutic agent for an allergic disease, said agent comprising, as a major component, an antibody that binds to one or more proteins selected from the group consisting of carboxypeptidase M, cathepsin C, endothelin-A receptor, osteoblast specific factor 2, DD96, and CYP1B1.
[15] Use of a transgenic non-human vertebrate in which the expression level of one or more genes selected from the group consisting of carboxypeptidase M, cathepsin C, endothelin-A receptor, osteoblast specific factor 2, DD96 (MAP17), and CYP1B1, and/or one or more genes functionally equivalent thereto is elevated in respiratory tract epithelial cells, as an animal model for an allergic disease.
[16] A kit for screening for a candidate compound for a therapeutic agent for an allergic disease, the kit comprising
   a polynucleotide that is at least 15 nucleotides long and that hybridizes to a nucleotide sequence of one or more genes selected from the group consisting of carboxypeptidase M, cathepsin C, endothelin-A receptor, osteoblast specific factor 2, DD96(MAP17), and CYP1B1 or to a complementary sequence thereof, and
   a cell that expresses a gene comprising a nucleotide sequence of one or more genes selected from the group consisting of carboxypeptidase M, cathepsin C, endothelin-A receptor, osteoblast specific factor 2, DD96 (MAP17), and CYP1B1.
[17] A kit for screening for a candidate compound for a therapeutic agent for an allergic disease, the kit comprising
   an antibody that recognizes a peptide having an amino acid sequence of one or more proteins selected from the group consisting of carboxypeptidase M, cathepsin C, endothelin-A receptor, osteoblast specific factor 2, DD96 (MAP17), and CYP1B1, and
   a cell that expresses a gene comprising a nucleotide sequence of one or more genes selected from the group consisting of carboxypeptidase M, cathepsin C, endothelin-A receptor, osteoblast specific factor 2, DD96(MAP17), and CYP1B1.

Otherwise, this invention relates to a method of treating an allergic disease, the method comprising the step of administering a compound that can be obtained by the method of any one of [7] , [9] , [10], and [11]. Furthermore, this invention relates to use of a compound that can be obtained by the method of any one of [7], [9], [10], and [11], for producing a pharmaceutical composition for treating an allergic disease. Additionally, this invention relates to a method of treating an allergic disease, the method including the step of administering an antisense DNA against the aforementioned gene or an antibody that binds to the aforementioned protein. Also, this invention relates to a use of an antisense DNA against the aforementioned gene or an antibody that binds to the aforementioned protein, for producing a pharmaceutical composition for treating an allergic disease.

In addition, the existence of all of these 6 genes has been elucidated. The nucleotide sequences of the 6 genes and the amino acid sequences encoded by them are shown in the following SEQ ID NOs

| | Nucleotide sequence | Amino acid sequence |
|---|---|---|
| Carboxypeptidase M | SEQ ID NO: 26 | SEQ ID NO: 27 |
| Cathepsin C | SEQ ID NO: 28 | SEQ ID NO: 29 |
| Endothelin-A receptor | SEQ ID NO: 30 | SEQ ID NO: 31 |
| Osteoblast specific factor 2 | | |
| | SEQ ID NO: 32 | SEQ ID NO: 33 |
| DD96 (MAP17) | SEQ ID NO: 34 | SEQ ID NO: 35 |
| CYP1B1 | SEQ ID NO: 36 | SEQ ID NO: 37 |

The already elucidated utility of the 6 genes used in this invention are shown below. For all of the genes, as indicated below, it was not known that their expression is enhanced in respiratory tract epithelial cells in response to IL-4 and IL-13.

### Carboxypeptidase M:

Regarding carboxypeptidase M, there is no report in particular regarding its utility.

### Cathepsin C:

A homologue of cathepsin C is shown to be useful as a diagnostic agent or a therapeutic agent for a monocytic and a macrophagic disease. Also, its use as a therapeutic agent or a diagnostic agent for type II collagen has been reported.

### Endothelin-A receptor:

It has been shown that this can be used for measuring endothelin, and for screening for antagonists against endothelin. Otherwise, it has been reported that this can be used as an inhibitor for an endothelin related disease.

### Osteoblast specific factor 2:

Its use as a diagnostic agent for bone metabolism, or as a tumor marker has been reported. Furthermore, regarding DNA and monoclonal antibodies, their use as therapeutic agents for thoracic, large intestine, or gastrointestinal cancer have been indicated. Also, use as an inhibitor, diagnostic agent, or therapeutic agent for heart, lung, and inflammatory diseases have been known.

### DD96 (MAP17):

It has been reported that 5'-EST of DD96 (MAP17) may be used for diagnosis, forensic medicine, gene therapy, and chromosome mapping. CYP1B1:

It has been shown that this can be used as a diagnostic marker for renal cancer and glaucoma.

In the present invention, allergic disease is a general term for diseases in which allergic reactions is involved. More specifically, for a disease to be considered allergic, the allergen must be identified, a strong correlation between exposure to the allergen and the onset of the pathological change must be demonstrated, and the pathological change has been proven to have an immunological mechanism. Herein, an immunological mechanism means that the leukocytes show an immune response to allergen stimulation. Examples of allergens are the mite antigen, pollen antigen, etc.

Representative allergic diseases are bronchial asthma, allergic rhinitis, pollinosis, insect allergy, etc. Allergic diathesis is a genetic factor that is inherited from allergic parents to children. Familial allergic diseases are also called atopic diseases, and their causative factor that can be inherited is atopic diathesis. Among atopic diseases, asthma is a general term for diseases accompanied with respiratory organ symptoms.

The method of testing for an allergic disease of this invention includes the steps of measuring the expression level of one or more genes selected from the group consisting of carboxypeptidase M, cathepsin C, endothelin-A receptor, osteoblast specific factor 2 (OSF2os), DD96 (MAP17), and CYP1B1 in a biological sample from a subject, and comparing the measured value with that for a normal healthy subject. As a result of comparing the two, if expression is enhanced compared to that of a normal healthy subject, the subject is judged to have an allergic disease. In this invention, the six aforementioned genes that can be indicators of allergic diseases are called indicator genes. Unless otherwise stated, the term "indicator gene" as used herein means any one or more genes selected from the six genes mentioned above.

In the method of testing for allergies based on this invention, indicator genes that are the target of expression level and activity measurements are selected from the six aforementioned indicator genes. Therefore, testing based on this invention can be performed by measuring the expression level and activity of any one of the genes of the 6 types of genes. Furthermore, in this invention, accuracy of the testing can be enhanced by measuring a plurality of these genes in combination. Since bronchial asthma patients form a heterogeneous group, more accurate diagnosis can be performed using a plurality of genes as indicators. Specifically, at least one, preferably 2 or more, more preferably 3 or more, and even more preferably 4 or 5 types or more indicator genes can be combined.

In this invention, expression levels of indicator genes include transcription of these genes to mRNA, and translation into proteins. Therefore, the method of testing for an allergic disease of this invention is performed based on comparing the strength of expression of mRNA corresponding to the aforementioned genes, or the expression level of proteins encoded by the aforementioned genes.

The measurement of expression level of indicator genes in the testing of allergic diseases of this invention can be carried out according to known genetic analysis methods. Specifically, one can use, for example, a hybridization technique that uses nucleic acids that hybridize to these genes as probes, or a gene amplification technique that uses DNAs that hybridize to the genes used in this invention as primers can be used.

The probes or primers used for the testing of this invention can be set based on the nucleotide sequences of the aforementioned indicator genes. The nucleotide sequences of the a forementioned indicator genes are well known. The GenBank accession numbers for the nucleotide sequence of each of the indicator genes are indicated in the Examples.

Genes of higher animals are generally accompanied by polymorphism in a high frequency. There exist many molecules that produce isoforms comprising different amino acid sequences from each other during the splicing process. Any genes associated with allergy which have a similar activity to that of the indicator gene are included in the indicator gene of the present invention, even though they carry mutation in the nucleotide sequence due to polymorphism and isoform.

As a primer or probe can be used a polynucleotide comprising the nucleotide sequence of the indicator gene or at least 15 nucleotides that are complementary to the complementary strand thereof. Herein, the term "complementary strand" means one strand of a double stranded DNA composed of A:T (U for RNA) and G:C base pairs to the other strand. In addition, "complementary" means not only those completely complementary to a region of at least 15 continuous nucleotides, but also having a homology of at least 70%, preferably at least 80%, more preferably 90%, and even more preferably 95% or higher. The degree of homology between nucleotide sequences can be determined by the algorithm, BLAST, etc.

Such polynucleotides are useful as the probe to detect an indicator gene, or as the primer to amplify the indicator gene. When used as a primer, those polynucleotides comprises usually 15 bp~ 100 bp, preferably 15 bp~ 35 bp of nucleotides. When used as a probe, DNAs comprising the whole sequence of the indicator gene (or a complementary strand thereof), or a partial sequence thereof that contains at least 15-bp nucleotides. When used as a primer, the 3' region thereof must be complementary to the indicator gene, while the 5'region can be linked to a restriction enzyme-recognition sequence or tag.

"Polynucleotides" in the present invention may be either DNA or RNA. These polynucleotides may be either synthetic or naturally-occurring. Also, DNA used as a probe for hybridization is usually labeled. Examples of labeling methods are those as described below. Herein, the term "oligonucleotide" means a polynucleotide with relatively low degree of polymerization. Oligonucleotides are included in polynucleotides. The labeling methods are as follows:
· nick translation labeling using DNA polymerase I;
· end labeling using polynucleotide kinase;
· fill-in end labeling using Klenow fragment (Berger, SL, Kimriel, AR. (1987) Guide to Molecular Cloning Techniques, Method in Enzymology, Academic Press; Hames, BD, Higgins, SJ (1985) Genes Probes: A Practical Approach. IRL Press; Sambrook, J, Fritsch, EF, Maniatis, T. (1989) Molecular Cloning: a Laboratory Manual, 2nd Edn. Cold Spring Harbor Laboratory Press) ;
· transcription labeling using RNA polymerase (Melton, DA, Krieg, PA, Rebagkiati, MR, Maniatis, T, Zinn, K, Green, MR. (1984) Nucleic Acid Res., 12, 7035-7056); and
· non-isotopic labeling of DNA by incorporating modified nucleotides (Kricka, LJ. (1992) Nonisotopic DNA Probing Techniques.

### Academic Press).

For testing for an allergic disease using hybridization techniques, for example, Northern hybridization, dot blot hybridization, or DNA microarray technique may be used. Furthermore, gene amplification techniques, such as RT-PCR method may be used. By using the PCR amplification monitoring method during the gene amplification step in RT-PCR, one can achieve more quantitative analysis for the gene expression of the present invention.

In the PCR gene amplification monitoring method, the detection target (DNA or reverse transcript of RNA) is hybridized to probes that are dual-labeled at both ends with different fluorescent dyes whose fluorescences cancel each other out. When the PCR proceeds and Taq polymerase degrades the probe with its 5'-3' exonuclease activity, the two fluorescent dyes become distant from each other and the fluorescence becomes to be detected. The fluorescence is detected in real time. By simultaneously measuring a standard sample in which the copy number of the target is known, it is possible to determine the copy number of the target in the subject sample with the cycle number where PCR amplification is linear (Holland, P. M. et al. , 1991, Proc. Natl. Acad. Sci. USA 88: 7276-7280; Livak, K. J. et al., 1995, PCR Methods and Applications 4 (6) : 357-362; Heid, C. A. et al., 1996, Genome Research 6: 986-994; Gibson, E. M. U. et al., 1996, Genome Research 6: 995-1001) . For the PCR amplification monitoring method, for example, ABI PRISM7700 (PE Biosystems) may be used.

The method of testing for an allergic disease in the present invention can be also carried out by detecting a protein encoded by the indicator gene. Hereinafter, a protein encoded by the indicator gene is described as an indicator protein. For such test methods, for example, Western blotting method, immunoprecipitation method, and ELISA method may be employed using antibody that binds to the indicator protein.

Antibodies that bind to the indicator protein used in the detection may be produced by techniques known to those skilled in the art. Antibodies used in the present invention may be polyclonal or monoclonal antibodies (Milstein, C. et al., 1983, Nature 305 (5934): 537-40). For example, polyclonal antibody against an indicator protein may be produced by collecting the blood from mammals sensitized with the antigen, and separating the serum from this blood using known methods. As a polyclonal antibody, the serum containing polyclonal antibody as such may be used. As the occasion demands, a fraction containing polyclonal antibody can be further isolated from this serum. Also, monoclonal antibody may be obtained by isolating immune cells from mammals sensitized with the antigen, fusing these cells with myeloma cells, and such, cloning hybridomas thus obtained, and collecting the antibody as a monoclonal antibody from the culture of the hybridomas.

For detecting an indicator protein, these antibodies may be appropriately labeled. Alternatively, instead of labeling the antibody, a substance that specifically binds to the antibody, for example, protein A or protein G, may be labeled to arrange an indirect detection of indicator protein. More specifically, one example of an indirect detection method is ELISA.

Protein or its partial peptide used as an antigen may be obtained, for example, by inserting the gene or its portion into an expression vector, introducing it into an appropriate host cell to produce a transformant, culturing the transformant to express the recombinant protein, and purifying the expressed recombinant protein from the culture or the culture supernatant. Alternatively, amino acid sequences encoded by these genes, or oligopeptides comprising portions of the amino acid sequence encoded by the full-length cDNA are chemically synthesized to be used as the antigen.

Furthermore, in the present invention, a testing for an allergic disease can be performed using not only the expression level of an indicator gene but also the activity of an indicator protein in the biological sample as an index. Activity of an indicator protein means a biological activity intrinsic to each protein. The detection of activity of an indicator protein can be achieved by known method.

Carboxypeptidase M (Tan, F.; Chan, S. J.; Steiner, D. F.; Schilling, J. W.; Skidgel, R. A.; Molecular cloning and sequencing of the cDNA for human membrane-bound carboxypeptidase M: comparison with carboxypeptidases A, B, H, andN. J. Biol. Chem. 264: 13165-13170, 1989) has been cloned from a cDNA library of human placenta, has an open reading frame of 1317 bp, and encodes 439 amino acids. It is expressed in many tissues and cultured cells as a membrane bound carboxypeptidase. It has been reported that this is expressed during differentiation from a monocyte to a macrophage, but its relation to allergic diseases is not known. Carboxypeptidase M is a protease that selectively cleaves basic amino acids at the C-terminal end. Therefore, based on this enzyme reaction, activity of carboxypeptidase M can be measured biologically. Examples of basic amino acids are arginine and lysine.

Cathepsin C (Paris, A.; Strukelj , B.; Pungercar, J.; Renko, M.; Dolenc, I.; Turk, V.: Molecular cloning and sequence analysis of human preprocathepsin C. FEBS Lett. 369: 326-330, 1995) is one of the proteases found in animal cells, also called dipeptidylpeptidase I. Its representative substrate is glycyl-L-phenylalaninamide. Therefore, the biological activity of cathepsin C can be found out by measuring the digestion activity of this substrate compound. Besides, cathepsin C is known to catalyze hydrolysis of an amide bond near pH 5 and to catalyze transfer reaction near pH 7 in which an amino group of a different substrate molecule is an acceptor.

Since endothelin-A receptors (Maggi, M.; Barni, T.; Fantoni, G.; Mancina, R. ; Pupilli, C.; Luconi, M.; Crescioli, C.; Serio, M.; Vannelli, G. B.; Expression and biological effects of endothelin-1 in human gonadotropin-releasing hormone-secreting neurons. J. Clin. Endocr. Metab. 85: 1658-1665, 2000) are receptor proteins, signal transduction into a cell due to binding of a ligand that can bind to these receptors can be referred to as the biological activity of each of these proteins. Signal transduction due to ligand binding can be detected by using as indicators, elevation of calcium concentration in a cell, change in cell morphology induced as a result of signal transduction, and such.

Osteoblast specific factor 2 (OSF2os; Horiuchi K, Amizuka N, Takeshita S, Takamatsu H, Katsuura M, Ozawa H, Toyama Y, Bonewald LF, Kudo A. ; Identification and characterization of a novel protein, periostin, with restricted expression to periosteum and periodontal ligament and increased expression by transforming growth factor beta. J Bone Miner Res. 1999 Jul; 14(7) :1239-49) is a 90 kDa protein. From differences in length at the C-terminal side due to alternative splicing, existence of 4 transcription products have been elucidated. It is now called periostin. It is expressed in the bones and some expression is found in the lungs. It is thought to be involved in cell adhesion during osteogenesis. It is highly homologous to betaig-h3, which is induced by TGFβ, and periostin itself is also induced by TGFβ in primary osteoblast cells. Its detection is possible using strengthened expression of bone specific genes as an indicator.

DD96 (Kocher O, Comella N, Tognazzi K, Brown LF.; Identification and partial characterization of PDZK1: a novel protein containing PDZ interaction domains. Lab Invest. 1998 Jan; 78 (1) :117-25) is a gene that is cloned from the kidney and encodes a 17 kD membrane bound protein. It is also called MAP17 and expression in cancer cells, cornified cells, and epithelial cells have been recognized, but its detailed function is not known.

CYP1B1 (Sutter, T. R.; Tang, Y. M.; Hayes, C. L.; Wo, Y.-Y. P.; Jabs, E. W.; Li, X.; Yin, H.; Cody, C. W.; Greenlee, W. F.: Complete cDNA sequence of a human dioxin-inducible mRNA identifies a new gene subfamily of cytochrome P450 that maps to chromosome 2. J. Biol. Chem. 269: 13092-13099, 1994) is one type of dioxin-inducible P450. P450 involved in metabolism of dioxins are involved in ring hydroxylation and cleavage, dehalogenation, glucuronidation, and such of the molecular structure of dioxin. Among them, CYP1B1 is a metabolic enzyme that metabolizes 2,3,7,8-TCDD. Examples of metabolites include a compound generated by Cl-elimination and hydroxylation of 2,3,7,8-TCDD. Therefore, CYP1B1 can be detected enzymatically using this enzyme activity as an indicator.

Normally, in the testing method of this invention, a biological sample from a subject is used as the sample. Respiratory tract epithelial cells and such may be used as the biological sample. Method to obtain respiratory tract epithelial cells is well known. That is, a sample can be obtained under a bronchoscope by physical detachment using forceps. The obtained sample is prepared by freezing, by formalin fixation, and by cultivation in a media. Furthermore, as the biological sample in the present invention, blood, sputum, secretion from nasal mucosa, bronchoalveolar lavage fluid, lung scrape, and such may be used. These biological samples are collected using known methods.

When the biological sample is cells of respiratory tract epithelial cells and such, samples for immunological measurements of the aforementioned proteins can be made by preparing a lysate. Otherwise, samples for measuring mRNA corresponding to the aforementioned genes can be made by extracting mRNA from this lysate. For extraction of lysate and mRNA of the biological sample, it is useful to utilize a commercially available kit. Otherwise, biological samples in the liquid form such as blood, nasal mucous secretion, and bronchoalveolar lavage fluid can be made into samples for measurement of proteins and genes by diluting with buffer and such, as necessary.

The measured value of expression level of indicator genes in respiratory tract epithelial cells can be corrected by known methods. As a result of correction, change in gene expression level in cells can be compared. Based on the measured value of expression level of genes that are expressed in the respiratory tract epithelial cells and do not show large fluctuations in their expression level regardless of the condition of the cell (housekeeping genes), correction of the measured value is performed by correcting the measured value of expression level of the genes that are to be used as indicators in this invention.

The indicator genes of this invention showed increase of expression level in a plurality of respiratory tract epithelial cell lines stimulated with IL-4 or IL-13. Therefore, testing for allergic diseases such as bronchial asthma can be performed using the expression level of indicator genes as indicators.

Test for an allergic disease in the present invention includes, for example, the tests as described below. Even a patient who, in spite of manifestation of bronchial asthma, can be hardly diagnosed with an allergic disease by conventional tests can be easily judged to be an allergic disease patient by carrying out the tests based on this invention. More specifically, the increase in the expression level of the indicator gene in a patient showing symptoms suspected of allergic disease indicates a high possibility that the symptoms are caused by an allergic disease. There are two types of bronchial asthma, one type being caused by an allergic reaction, and the other type not. Since treatments for two types are completely different, diagnosis as to which type causes the bronchial asthma is a very important step in the treatment. The test method of this invention can provide an extremely important information in identifying causes of bronchial asthma.

Otherwise, tests to judge whether the allergic symptom is improving become possible. The indicator genes of this invention showed increase in expression level in the respiratory tract epithelial cells stimulated with IL-4 or IL-13. In bronchial asthma, respiratory tract epithelial tissue is a tissue that shows remarkable lesion. Therefore, genes whose expression varies in respiratory tract epithelial cells simulated with IL-4 or IL-13, which are cytokines that strongly induce allergic reactions, are useful for judging therapeutic effects. More specifically, elevation of expression of indicator genes in patients diagnosed with an allergic disease indicates that there is a strong possibility that the allergic symptom is progressing.

Additionally, this invention relates to use of a transgenic non-human animal in which expression level of indicator genes in the respiratory tract epithelial cells is elevated, as an animal model for an allergic disease. Animal model for an allergic disease is useful for elucidating changes of bronchial asthma in vivo. Furthermore, the animal model for an allergic disease of this invention is useful for evaluating therapeutic agents for allergic bronchial asthma.

Because of this invention, it was elucidated that the expression levels of the aforementioned indicator genes in respiratory tract epithelial cells rise due to stimulation by IL-4 or IL-13 . Therefore, animals in which expression levels of these genes or genes functionally equivalent to these genes are artificially enhanced in respiratory tract epithelial cells can be used as animal models for allergic diseases. Elevation of expression level in respiratory tract epithelial cells includes elevation of expression level of target genes in the entire respiratory tract tissue. That is, elevation of expression level of the aforementioned genes includes elevation of the expression level of the genes in the entire respiratory tract tissue or in the entire body, in addition to the elevation in the respiratory tract epithelia alone.

The functionally equivalent genes used in this invention are genes that encode proteins having activity similar to the known activity of proteins encoded by each of the indicator genes. A representative example of functionally equivalent genes includes a counterpart of indicator genes of a transgenic animal that intrinsically has the counterpart.

Otherwise, genes that encode proteins having for example, sequence homology of 90% or more, preferably 95% or more, and more preferably 99% or more, towards amino acid sequence of the aforementioned indicator proteins can be shown to be genes that are functionally equivalent to the aforementioned indicator genes. Additionally, genes (1) that can be amplified using, as primers, oligonucleotides comprising the nucleotide sequence of carboxypeptidase M, cathepsin C, endothelin-A receptor, osteoblast specific factor 2, DD96 (MAP17), and CYP1B1, which were used in the Examples, and (2) that encode proteins whose expression level significantly increases in respiratory tract epithelial cells stimulated with IL-4 and IL-13 are functionally equivalent genes.

Genes whose expression increase due to IL-4 or IL-13 stimulation can be said to be genes included in the signal transduction pathway of these cytokines. In other words, IL-4 or IL-13 stimulation can be considered to be expressed as allergic symptoms through enhancement of expression of these genes. That is, genes that increase their expression due to IL-4 or IL-13 stimulation can be said to be genes that accomplish important roles in pathological formation of allergies in the respiratory tract epithelium. Therefore, in treatment of allergies, drugs that suppress the expression of these genes or inhibit their activities are expected to have the effect of not only simply improving allergic symptoms but also removing the fundamental cause of pathological formation of allergies.

As described above, a gene the expression level of which in respiratory tract epithelial cells is increased upon stimulation thereof with IL-4 or IL-13 is very important. Therefore, it is highly significant to assess the role of the gene and effects of drugs targeting this gene using transgenic animals, which can be obtained by elevating the expression level of this gene *in vivo,* as the allergic disease model animal.

Allergic disease model animals according to the present invention are useful in not only screening for drugs for treating or preventing allergic diseases as described below but also elucidating mechanisms of allergic diseases, furthermore, testing the safety of compounds screened.

For example, if allergic disease model animals according to the present invention either develop clinical manifestations of bronchial asthma or show changes in measured values related to any allergic diseases, it is possible to construct a screening system for searching for a compound having activity to recover normal conditions.

In the present invention, increase in the expression level means the state wherein a target gene is transduced as a foreign gene and forcibly expressed; the state wherein transcription of a gene inherent in the host and translation thereof into protein are increased; or the state wherein decomposition of the translation product, protein, is suppressed. Gene expression level can be confirmed by, for example, the quantitative PCR as described in Examples. Furthermore, activity of translation product, protein, can be confirmed by comparing to that in the normal state.

A typical transgenic animal is the one to which a gene of interest is transduced to be forcibly expressed. Examples of another type of transgenic animals are those in which a mutation is introduced into the coding region of the gene to increase its activity or to modify the amino acid sequence of the gene product protein so as to be hardly decomposed. Examples of mutation in the amino acid sequence are the substitution, deletion, insertion, or addition of amino acid(s). In addition, by mutagenizing the transcriptional regulatory region of the gene, the expression itself of the gene of this invention can be controlled.

Methods for obtaining transgenic animals with a particular gene as a target are known. That is, a transgenic animal can be obtained by a method wherein the gene and ovum are mixed and treated with calcium phosphate; a method wherein the gene is introduced directly into the nucleus of oocyte in pronuclei with a micropipette under a phase contrast microscope (microinjection method, US Patent No. 4873191) ; or a method wherein embryonic stem cells (ES cells) are used. Furthermore, there have been developed a method for infecting ovum with a gene-inserted retrovirus vector, a sperm vector method for transducing a gene into ovum via sperm, or such. Sperm vector method is a gene recombination technique for introducing a foreign gene by fertilizing ovum with sperm after a foreign gene has been incorporated into sperm by the adhesion or electroporation method, etc. (M. Lavitranoet, et al. Cell, 57, 717, 1989).

Transgenic animals used as the allergic disease model animal of the present invention can be produced using all the vertebrates except for humans. More specifically, transgenic animals having various transgene and being modified gene expression levels thereof are produced using vertebrates such as mice, rats, rabbits, miniature pigs, goats, sheep, or cattle.

Furthermore, the present invention relates to a method of screening for a candidate compound for a therapeutic agent for an allergic disease. In this invention, the indicator gene shows a significant increase in its expression level in respiratory tract epithelial cells stimulated with IL-4 or IL-13. Therefore, it is possible to obtain a therapeutic agent for an allergic disease by selecting a compound capable of reducing the expression level of such a gene. Compounds that reduce the expression level of a gene are those having inhibitory effects on any steps of the transcription or translation of a gene, or the activity expression of a protein.

A method of screening for a therapeutic agent for an allergic disease of this invention can be carried out either *in vivo* or *in vitro.* This screening method can be carried out, for example, according to the steps as described below. The indicator gene in the screening method of this invention includes, in addition to the genes described as indicator genes above, any genes functionally equivalent thereto. The steps of the screening method are:
(1) administering a candidate compound to a test animal;
(2) measuring the expression level of the indicator gene in a biological sample from the test animal; and
(3) selecting a compound that reduces the expression level of the indicator gene, compared to a control.

As a test animal in the screening method of the present invention, for example, an allergic disease model animal, a transgenic animal in which a human indicator gene is forcibly expressed, may be used. When a promoter whose transcription activity is controlled by a substance such as an appropriate drug is used in the expression vector, the expression level of the foreign indicator gene can be adjusted by administering said substance to the transgenic animal.

Thus, by administering a drug candidate compound to a model animal in which an indicator gene is forcibly expressed, and monitoring the action of the compound toward the expression of the indicator gene in the biological sample from the model animal, effects of the drug candidate compound on the expression level of the indicator gene can be detected. Changes in the expression levels of the indicator gene in biological samples from test animals can be monitored by a method similar to above-described testing method of this invention. Furthermore, based on the detection results, by selecting compounds that reduce the expression level of the indicator gene, drug candidate compounds can be screened.

More specifically, the screening according to the present invention can be carried out by comparing the expression level of the indicator gene in the biological sample collected from a test animal to that in a control. As a biological sample, smooth muscle cells, cornified cells, nasal mucosa epithelial cells, intestinal epithelial cells, lymphocytes, mast cells, eosinophils, basophils, neutrophils, and such can be used. Methods for collecting and preparing these biological samples are known.

These screening methods enable the selection of drugs involved in the expression of indicator genes in various ways. More specifically, for example, drug candidate compounds having the following action points can be found:
activation of signal transduction pathway to induce the expression of an indicator gene;
elevation of the transcription activity of the indicator gene; and
stabilization of the transcription product of the indicator gene or inhibition of the decomposition thereof, etc.

Examples of *in vitro* screening include a method in which cells expressing an indicator gene are contacted with a candidate compound to select a compound that reduces the expression level of the indicator gene. This screening may be carried out, for example, according to the steps of:
(1) contacting a candidate compound with cells expressing an indicator gene;
(2) measuring the expression level of the indicator gene; and
(3) selecting a compound that reduces the expression level of the candidate gene, compared to a control.

In the present invention, cells expressing an indicator gene can be obtained by inserting the indicator gene to an appropriate expression vector, and introducing said vector into a suitable host cell. Any vectors and host cells may be used as long as they are able to express the gene of this invention. Examples of host cells in the host-vector system are *Escherichia coli*, yeast, insect cells, animal cells, and such, and vectors usable for respective host cells can be appropriately selected.

Vectors may be introduced into the host by the biological method, physical method, chemical method, etc. Examples of the biological method are a method using virus vectors, a method using a specific receptor, cell-fusion method (HVJ (Senda virus) method, polyethylene glycol (PEG) method, electric cell fusion method, microcell-mediated chromosome transfer. Examples of the physical method are a microinjection method, electroporation method, and a method using the gene particle gun (gene gun). Examples of the chemical method are a calcium phosphate precipitation method, liposome method, DEAE-dextran method, protoplast method, erythrocyte ghost method, erythrocyte membrane ghost method, and microcapsule method.

As cells in which an indicator gene expresses, the human lung cancer cell A549 and human bronchial epithelial cell BEAS-2B are preferable for the screening method of the present invention. These cells are commercially available from ATCC.

In the screening method of this invention, first a candidate compound is added to the cell strain. Then, the expression level of an indicator gene in the cell strain is measured to select a compound that reduces the expression level of the gene.

In the screening method of this invention, expression levels of indicator genes can be compared not only based on the expression levels of proteins encoded by these genes but also based on the corresponding mRNAs detected. For performing the comparison of expression levels using mRNA, the process for preparing mRNA sample as described above is carried out in place of the process for preparing protein samples. Detection of mRNA and protein can be performed by known methods as described above.

Furthermore, based on the disclosure of this invention, it is possible to obtain the transcriptional regulatory region for the indicator gene of this invention and construct a reporter assay system. Reporter assay system means a system for screening for a transcriptional regulatory factor that acts on the transcriptional regulatory region using the expression level of a reporter gene localized downstream of the transcriptional regulatory region as an index.

That is, this invention relates to a method of screening for candidate compounds for a therapeutic agent for an allergic disease, in which the indicator genes are one or more genes selected from the group consisting of carboxypeptidase M, cathepsin C, endothelin-A receptor, osteoblast specific factor 2, DD96(MAP17), CYP1B1, and genes that are functionally equivalent to these genes, the method comprising the following steps:
(1) contacting a candidate substance with cells transfected with a vector containing a transcription regulatory region of one or more indicator genes and a reporter gene that is expressed under the control of the transcription regulatory region,
(2) measuring the activity of the aforementioned reporter gene, and
(3) selecting a compound that lowers the reporter gene expression level, compared to a control.

Examples of transcription regulatory regions are promoters, enhancers, and furthermore, CAAT box and TATA box, which are normally seen in the promoter region. Also, as reporter genes, CAT (chloramphenicol acetyltransferase) gene, luciferase gene, growth hormone genes, and such may be used. In not a few number of the indicator genes of this invention, a transcription regulatory region has already been elucidated.

Otherwise, the transcription regulatory region of the indicator genes of this invention can be obtained as follows. That is, first, screening is performed by a method that uses PCR or hybridization based on the nucleotide sequences of the indicator genes disclosed in this invention, and a genomic DNA clone containing the cDNA sequence is obtained from a human genome DNA library such as BAC library and YAC library. Based on the obtained genomic DNA sequence, the transcription regulatory region of cDNA disclosed in this invention is estimated, and the transcription regulatory region is obtained. A reporter construct is constructed by cloning the obtained transcription regulatory region so that it is positioned upstream of the reporter gene. The obtained reporter construct is transfected into a cultured cell strain and is made into a transformant for screening. By contacting the candidate compounds with this transformant, screening of compounds that regulate the expression of reporter genes can be performed.

As the screening method of this invention *in vitro*, a screening method based on activities of indicator proteins can be used. That is, this invention relates to a method of screening for a therapeutic agent for an allergic disease, in which the indicator genes are one or more genes selected from the group consisting of carboxypeptidase M, cathepsin C, endothelin-A receptor, osteoblast specific factor 2, DD96 (MAP17), CYP1B1, and genes that are functionally equivalent to these genes, the method comprising the following steps:
(1) contacting the candidate substance with one or more proteins encoded by the indicator genes,
(2) measuring the activity of the aforementioned proteins,
(3) selecting a compound that lowers the activity of the aforementioned proteins, compared to a control.

Each of the activities possessed by the indicator proteins of this invention, which are carboxypeptidase M, cathepsin C, endothelin-A receptor, osteoblast specific factor 2, DD96 (MAP17), and CYP1B1, has been already mentioned. Using these activities as indicators, compounds having activity to inhibit these activities can be screened. Compounds that can be obtained in this manner suppress the function of carboxypeptidase M, cathepsin C, endothelin-A receptor, osteoblast specific factor 2, DD96(MAP17), and CYP1B1. As a result, bronchial asthmatic attack can be regulated through inhibition of indicator proteins whose expression is induced in the respiratory tract epithelial cells.

Candidate test compounds used in such screening include, in addition to compound preparations synthesized by existing chemical methods such as steroid derivatives and compound preparations synthesized by combinatorial chemistry, mixtures of multiple compounds such as extracts from animal or plant tissues, or microbial cultures, and their purified preparations, etc.

Polynucleotide, antibody, cell strain, or model animal necessary for various screening methods according to this invention can be previously combined into a kit. More specifically, for example, a kit may be composed of a cell expressing an indicator gene and a reagent to measure the expression level of the indicator gene. As a reagent for measuring the expression level of an indicator gene, for example, a polynucleotide containing the nucleotide sequence of at least one indicator gene, or an at least 15-nucleotide-long oligonucleotides containing a nucleotide sequence complementary to the complementary strand thereof can be used. Alternatively, antibody that recognizes a peptide containing the amino acid sequence of at least one indicator protein may be used as a reagent. In these kits may be packaged a substrate compound used for the detection of the indicator, medium and vessel for cell culturing, positive and negative standard samples, and furthermore, a manual describing how to use the kit

The compounds selected by the screening method of this invention are useful as a therapeutic agent for an allergic disease. Also, the antisense DNA that can suppress the expression of an indicator gene, and, furthermore, antibody recognizing a protein encoded by an indicator gene are also useful as the therapeutic agent for an allergic disease. The therapeutic agent for an allergic disease according to this invention can be formulated by including the compound selected by the screening method as the effective ingredient, and mixing with a physiologically acceptable carrier, excipient, diluent, or the like. Aiming at the amelioration of allergic symptoms, the therapeutic agent for an allergic disease of this invention can be administered orally or parenterally.

Oral drugs can take any dosage forms selected from a group of granule, powder, tablet, capsule, solution, emulsion, suspension, etc. Injections can include the subcutaneous injection, intramuscular injection, intraperitoneal injection, etc.

Furthermore, for administering the compound that is composed of protein, the therapeutic effect can be achieved by introducing a gene encoding the protein into the living body using gene therapeutic techniques. The techniques for treating disease by introducing a gene encoding a therapeutically effective protein into the living body and expressing it therein are known.

Alternatively, the antisense DNA can be incorporated downstream of an appropriate promoter sequence to be administered as an antisense RNA expression vector. When this expression vector is introduced into T cells of an allergic disease patient, the therapeutic effect on allergic disease can be achieved by reducing the expression level of the gene through the expression of corresponding antisense gene. For introducing the expression vector into T cells, methods performed either *in vivo* or *ex vivo* are known.

Although the dosage may vary depending on the age, sex, body weight, and symptoms of a patient, treatment effects, method for administration, treatment duration, type of active ingredient contained in the drug composition, or such, it can be usually administered in the range of 0.1 mg~ 500 mg, preferably 0.5 mg~ 20 mg per dose for an adult. However, since the dosage varies according to various conditions, amount less than the above-described dosage may be sufficient in some cases, and dosage exceeding the above-described range may be required in other cases.

Any prior art literatures cited herein are incorporated by reference.

### Brief Description of the Drawings

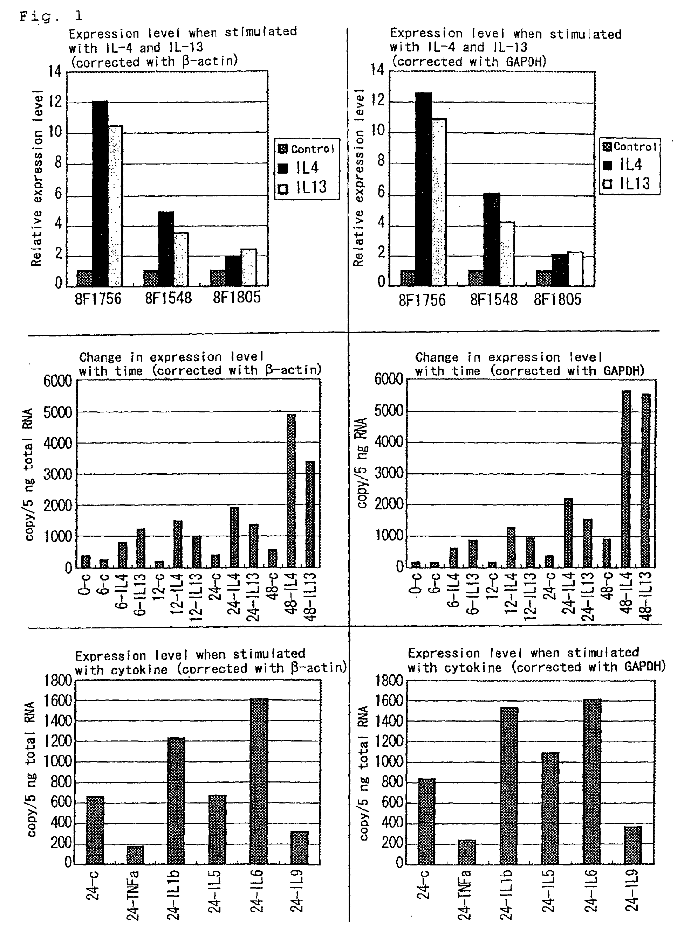

Fig. 1 is a set of graphs showing the results of measuring the expression levels of the carboxypeptidase M gene in cultured bronchial epithelial cells stimulated with IL-4 and IL-13, or with other cytokines. Those on the left are results corrected with β-actin gene, and those on the right are results corrected with GAPDH gene. Graphs on the top row indicate the relative expression level (relative ratio of control) in each lot of cells when stimulated with IL-4 (center) and IL-13 (right) to the control (left). Graphs in the middle row show change in expression level with time, 0, 6, 12, 24, and 48 hours after treatment. The numbers on the abscissa indicate cultivation time. Graphs in the bottom row show the change in expression level 24 hours after treatment with other cytokines.
Fig. 2 is a set of graphs showing the results of measuring the expression levels of the cathepsin C gene in cultured bronchial epithelial cells stimulated with IL-4 and IL-13, or with other cytokines. Each graph shows analogous content to that of each graph in Fig. 1.
Fig. 3 is a set of graphs showing the results of measuring the expression levels of the endothelin-A receptor gene in cultured bronchial epithelial cells stimulated with IL-4 and IL-13, or with other cytokines. Each graph shows analogous content to that of each graph in Fig. 1.
Fig. 4 is a set of graphs showing the results of measuring the expression levels of the osteoblast specific factor gene in cultured bronchial epithelial cells stimulated with IL-4 and IL-13, or with other cytokines. Each graph shows analogous content to that of each graph in Fig. 1.
Fig. 5 is a set of graphs showing the results of measuring the expression levels of the DD96 (MAP17) gene in cultured bronchial epithelial cells stimulated with IL-4 and IL-13, or with other cytokines. Each graph shows analogous content to that of each graph in Fig. 1.
Fig. 6 is a set of graphs showing the results of measuring the expression levels of the CYP1B1 gene in cultured bronchial epithelial cells stimulated with IL-4 and IL-13, or with other cytokines. Each graph shows analogous content to that of each graph in Fig. 1.

### Best Mode for Carrying out the Invention

The present invention will be explained in detail below with reference to examples, but it is not to be construed as being limited thereto.

### [Example 1] Selection of candidate genes using DNA microarray

### 1. Cultivation of normal human bronchial epithelial cells, and IL-4 or IL-13 stimulation

Three lots of normal human bronchial epithelial cells available from Clonetics were purchased (8F1756, 8F1548, 8F1805). Cells (5x 10⁵) contained in one vial were divided into three equal parts (1.67x 10⁵/75 cm² flask) for no stimulation, IL-4 stimulation, and IL-13 stimulation, and these were cultivated for approximately 8 to 10 days in SABM media (Clonetics) with medium exchange. During this procedure, BPE (bovine pituitary extract), hydrocortisone, hEGF, epinephrine, transferrin, insulin, retinoic acid, BSA-FAF, triiodothyronine, GA-1000 (gentamicin/amphotericin-B) were added to the media according to the attached protocol.

Before cytokine stimulation, the cells were washed with PBS, and then placed into SABM without added factors. IL-4 (10 ng/mL) and IL-13 (50 ng/mL) (both from Peprotech) were added thereto, and this was cultivated for 24 hours. Observation of changes with passage of time (0, 6, 12, 24, and 48 hours) was carried out in a similar manner.

### 2. Other cytokine stimulation of normal human bronchial epithelial cells

Using cells from lot 8F1548, cultivation was performed similarly to that of 1. In place of IL-4 and IL-13, 50 ng/mL of TNFα, IL-1β, IL-5, IL-6, and IL-9 (all from Peprotech) were added and cultivated for 24 hours.

### 3. Preparation of RNA for GeneChip

Respiratory tract epithelial cells treated as mentioned above were dissolved in Isogen (Nippon Gene; Wako Pure Chemicals) , and from this solution, RNA was separated according to a protocol attached to Isogen. After addition of chloroform, this was agitated, then centrifuged, and its aqueous layer was collected. Next, isopropanol was added, this was agitated, and then centrifuged to collect the precipitated total RNA.

### 4. cDNA synthesis for GeneChip

Single stranded cDNA was prepared from 5 µg of total RNA, which was prepared from cells of lot 8F1756, by reverse transcription using Superscript II Reverse Transcriptase (Life Technologies) following the method of Expression Analysis Technical Manual by Affymetrix, and by using T7-(dT)₂₄ (Amersham Pharmacia) as a primer. The T7- (dT) ₂₄ primer comprises a nucleotide sequence in which d(T)₂₄ is added to a T7 promoter nucleotide sequence, as shown below.
T7-(dT)₂₄ primer (SEQ ID NO: 1)
5'-GGCCAGTGAATTGTAATACGACTCACTATAGGGAGGCGG-(dT) ₂₄-3 '

Next, according to Expression Analysis Technical Manual, DNA ligase, DNA polymerase I, and RNase H were added to synthesize double stranded cDNA. After phenol-chloroform extraction of cDNA, this was passed through Phase Lock Gels, and was purified by ethanol precipitation.

Furthermore, using BioArray High Yield RNA Transcription Labeling Kit, biotin-labeled cRNA was synthesized. Using RNeasy Spin column (QIAGEN), cRNA was purified and then fragmented by treatment with heat.

To a hybridization cocktail, 12.5 µg of this cRNA was added according to Expression Analysis Technical Manual. This was placed into an array and was hybridized for 16 hours at 45°C.

After the array was washed, streptavidin phycoerythrin was added for staining. After washing, a mixed antibody solution of normal goat IgG and biotinylated goat IgG was added to the array. Furthermore, in order to enhance fluorescence intensity, streptavidin phycoerythrin was added again for staining. After washing, this was set into a scanner and was analyzed by a GeneChip software.

### 5. GeneChip analysis

Data analysis was performed using Suite, which is a GeneChip analysis software. Average Intensity (1) and Background Average (2) were investigated by Absolute Analysis, and 3 average values obtained for no stimulation, IL-4 stimulation, and IL-13 stimulation by subtracting (2) from (1) were used as scale factors for comparison analysis.

First, absolute analysis was performed to analyze one chip data. Positives and negatives were determined by comparing the fluorescence intensity of perfect match and mismatch of a probe set. Judgment into three categories of Absolute Calls, which are P (present) , A (absent) , and M (marginal), were made by values of Pos Fraction, Log Avg, and Pos/Neg.
Pos Fraction; ratio of positive pairs.
Log Avg; average of the log of fluorescence intensity ratio between probe cells of perfect match and mismatch.
Pos/Neg; ratio of the number of positive pairs and negative pairs.

Additionally, Average Difference (Avg Diff), which is the average value of the difference in fluorescence intensities between probe cells of perfect match and mismatch, was calculated for each gene.

Next, Comparison Analysis was performed on two sets of data. Comparisons were made between no stimulation and IL-4 stimulation, or between no stimulation and IL-13 stimulation, and differences in expression levels were ranked as follows. Judgment into 5 categories of difference calls, which are I, D, MI, MD, and NC, were made from values of Inc/Dec, Inc Ratio, Dpos-Dneg Ratio, and Log Avg Ratio Change.
Inc: Number of probe pairs that corresponded to IL-4 stimulation or IL-13 stimulation and no stimulation and that were judged to show increased expression levels for IL-4 stimulation or IL-13 stimulation.
Dec: Number of pairs judged to show decreased expression levels for IL-4 stimulation or IL-13 stimulation.
Inc/Dec: Ratio of the number of pairs judged to be Inc and number of pairs judged to be Dec.
Inc Ratio: Number of pairs judged to be Inc/number of pairs actually used.
Dpos/Dneg Ratio: Ratio between the number of Neg Change subtracted from that of Pos Change, and the number of pairs actually used.
Pos Change: Difference between the number of positive pairs in Absolute Analysis of IL-4 simulation or IL-13 stimulation, and the number of positive pairs in Absolute Analysis of no stimulation.
Neg Change: Difference between the number of negative pairs in Absolute Analysis of IL-4 simulation or IL-13 stimulation, and the number of negative pairs in Absolute Analysis of no stimulation.
Log Avg Ratio Change: Difference between Log Avg in Absolute Analysis of IL-4 stimulation or IL-13 stimulation and no stimulation.
- Increased:: I,
- Decreased:: D,
- Marginally Increased:: MI,
- Marginally Decreased:: MD, and
- No Change:: NC

Additionally, genes whose expression was altered (enhanced or reduced) due to IL-4 simulation or IL-13 stimulation by values of Fold Change, which is a ratio of Avg Diff in Absolute Analysis of no stimulation with IL-4 simulation or no stimulation with IL-13 stimulation, were selected. Table 1 shows the results of narrowing down expressed genes in respiratory tract epithelial cells by lot number.

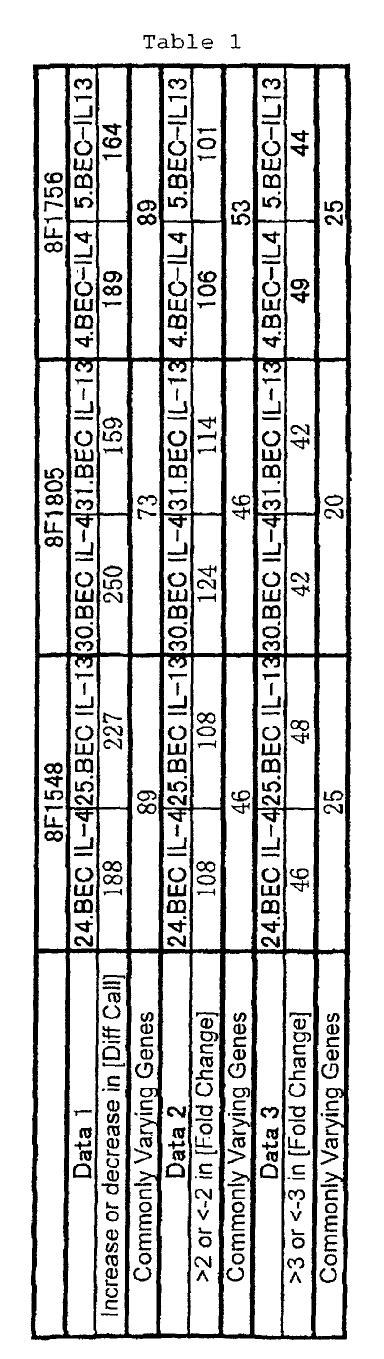

In the table, "increase or decrease in [Diff Call]" shows the number of genes whose expression levels showed differences due to IL-4 stimulation or IL-13 stimulation. The genes whose expression levels showed differences include genes that were judged as marginally increased (or marginally decreased) in the aforementioned ranking. The number of genes in which differences were commonly seen with both IL-4 and IL-13 was indicated for each lot as "Commonly Varying Genes". Furthermore, the number of genes in which increase or decrease of twofold or more (>2 or <-2), and increase or decrease of threefold or more (>3 or <-3) were observed was indicated as "[Fold Change]".

As a result, the following 6 types of genes were selected as genes whose expression levels showed twofold or greater increase for both IL-4 stimulation and IL-13 stimulation and whose expression levels commonly showed increase for two or more lots. These genes are closely related to allergies, and their expression levels increase by twofold or more due to stimulation by both IL-4 and IL-13, which are allergy related cytokines.
carboxypeptidase M
cathepsin C
endothelin-A receptor
osteoblast specific factor 2 (OSF2os)
DD96 (MAP17)
and dioxin-inducible cytochrome P450 (CYP1B1)

### [Example 2] Confirmation of expression level of candidate genes

To quantitatively confirm the expression level of 6 genes selected in Example 1, cultivated respiratory tract epithelial cells (Clonetics) were used to further perform quantitative PCR by ABI 7700. Three lots, 8F1756, 8F1548, and 8F1805, were used for the cultivated cells. Primer and TaqMan probe used for measurements by ABI 7700 were designed by Primer Express (PE Biosystems) based on sequence information of each gene. The 5'-end of TaqMan probe is labeled with FAM (6-carboxy-fluorescein) and the 3'-end is labeled with TAMRA (6-carboxy-N,N,N',N'-tetramethylrhodamine). Nucleotide sequences of oligonucleotides used for forward primer (F) , reverse primer (R) , and TaqMan probe (TP) of each gene are as shown below. Genbank Accession No. corresponding to the nucleotide sequence of each indicator gene is shown in parenthesis following the name.
Carboxypeptidase M (j04970) F: acagagacgtttgtcctctctgc (SEQ ID NO: 2) R: atgccccagttgcttgaacac (SEQ ID NO: 3) TP: ccctcgtggccagttacccatttgata (SEQ ID NO: 4) Cathepsin C (x87212) F: tctcagaccccaatcctaagcc (SEQ ID NO: 5) R: ctgcaataaggtatgggaagcc (SEQ ID NO: 6) TP: tcttgtagccagtatgctcaaggctgtgaa (SEQ ID NO: 7) Endothelin-A receptor (d11151) F: acctctgcgctcttagtgttgac (SEQ ID NO: 8) R: gcagttaccaaaggaatcccaa (SEQ ID NO: 9) TP: tacagagcagttgcctcctggagtcgtgtt (SEQ ID NO: 10) Osteoblast specific factor 2 (d13666) F: agcaaaccaccttcacggatc (SEQ ID NO: 11) R: ggtgccagcaaagtgtattctcc (SEQ ID NO: 12) TP: aattaggcttggcatctgctctgaggcc (SEQ ID NO: 13) DD96(MAP17) (u21049) F: gcctttgcagtcaaccacttctg (SEQ ID NO: 14) R: tctgttcccaccaggactccat (SEQ ID NO: 15) TP: atgatcctgaccgtcggaaacaaggc (SEQ ID NO: 16) CYP1B1(u03688)

F: ttatgaagccatgcgcttct (SEQ ID NO: 17)

R: aagacagaggtgttggcagtg (SEQ ID NO: 18)

TP: cagctttgtgcctgtcactattcctcatg (SEQ ID NO: 19)

Total RNA extracted by the aforementioned method was treated with DNase (Nippon Gene). Then, cDNA, which was reverse transcribed using random hexamer (GIBCO BRL) as primer, was used as a template. For a standard curve to calculate the number of copies, a plasmid clone containing a nucleotide sequence region that is amplified by both primers was prepared for each of the genes, and this was diluted stepwise to be used as template for carrying out the reaction. The composition of reaction solution for monitoring PCR amplification is shown in Table 2.

**Table 2**

| Composition of reaction in ABI-PRISM 7700 (Amount per well) | |
|---|---|
| Sterilized distilled water | 23.75 (µL) |
| 10x TaqMan buffer A | 5 |
| 25mM MgCl₂ | 7 |
| dATP (10 mM) | 1.0 |
| dCTP(10 mM) | 1.0 |
| dGTP(10 mM) | 1.0 |
| dUTP(20 mM) | 1.0 |
| Forward Primer (10 µM) | 1.0 |
| Reverse Primer (10 µM) | 1.0 |
| TaqMan probe (2.0 µM) | 2.5 |
| AmpliTaq Gold (5 U/µL) | 0.25 |
| AmpErase UNG (1 U/µL) | 0.5 |
| Template solution | 5 |
| Total | 50 |

Additionally, to correct the differences of cDNA concentration in the sample, similar quantitative analysis was performed for β-actin gene and glyceraldehyde-3-phosphate dehydrogenase (GAPDH) gene as internal standards for correction. By correcting based on the number of copies of these genes, the number of copies of the genes of interest was calculated.

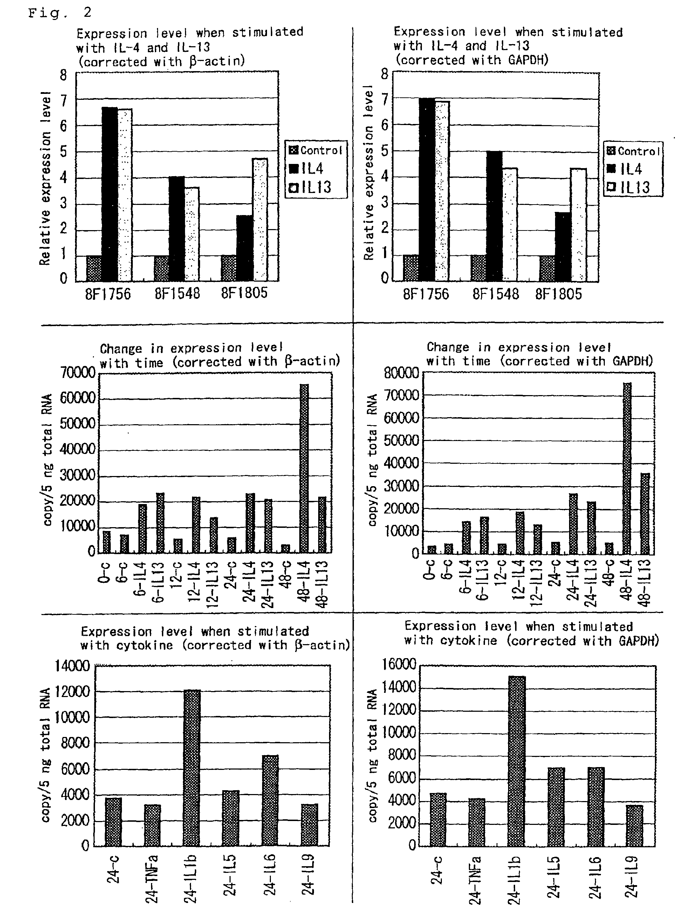

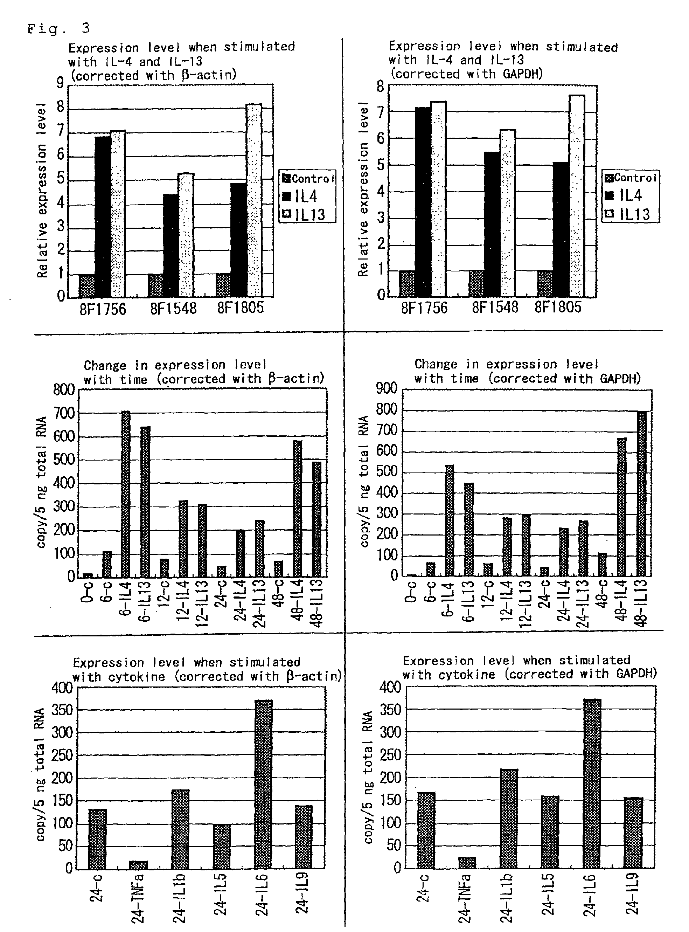

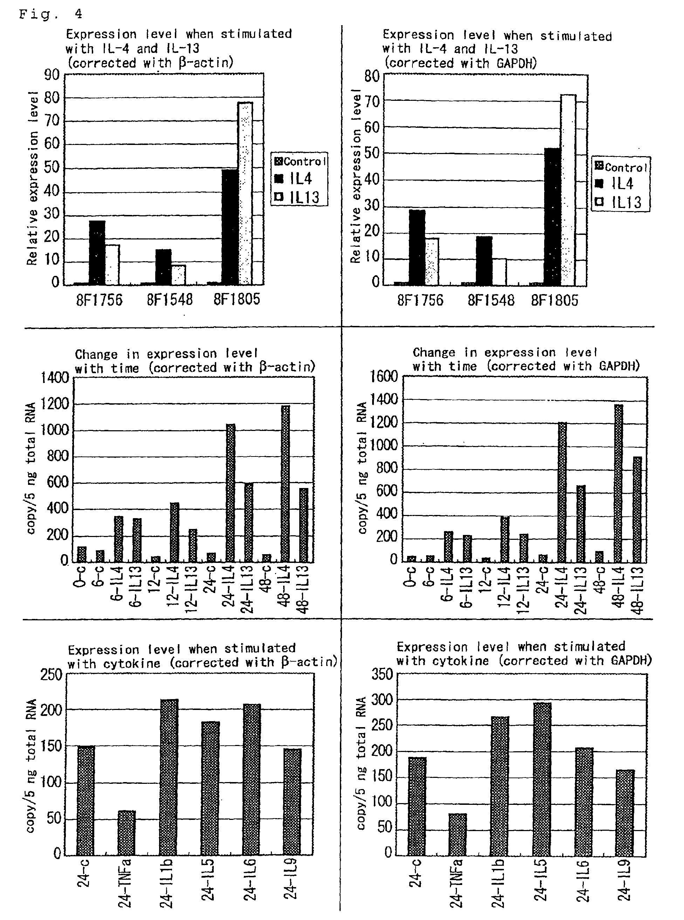

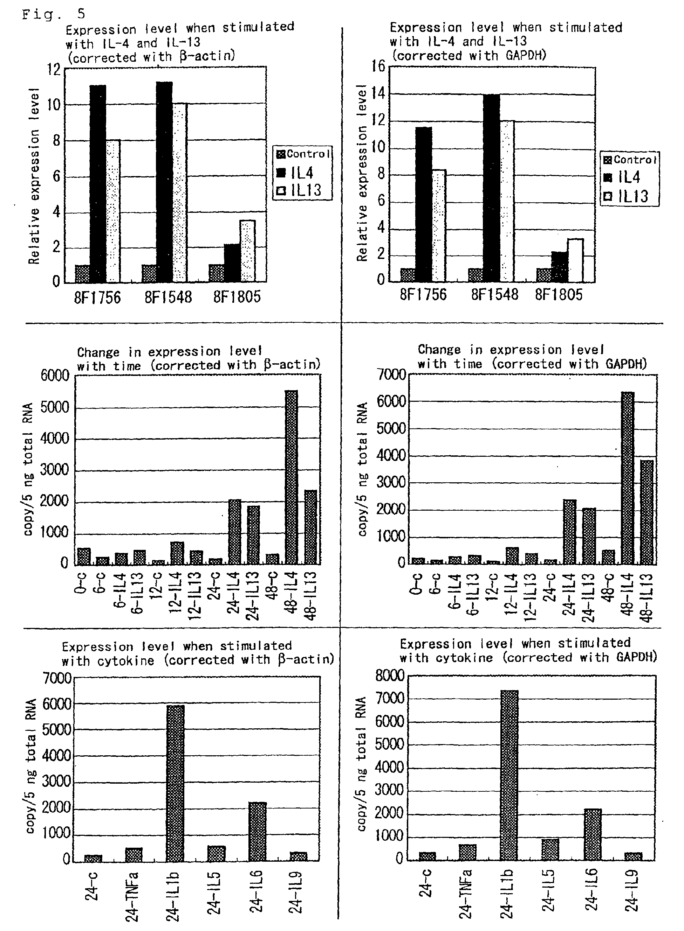

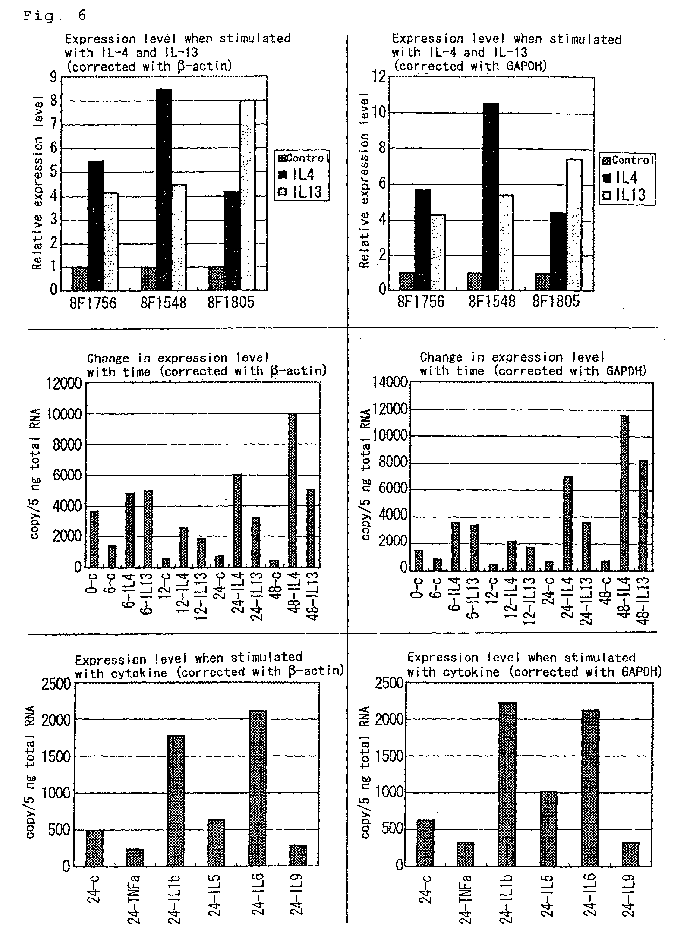

Primers and probes for measuring β-actin or GAPDH were those packaged with TaqMan β-actin Control Reagents (PE Biosystems). The nucleotide sequences are as shown below. The β-actin-corrected expression levels (copy/5 ng RNA) for each of the genes are shown in Figs. 1 to 6. 
β-actin forward primer (SEQ ID NO: 20)
TCA CCC ACA CTG TGC CCA TCT ACG A
 
β-actin reverse primer (SEQ ID NO: 21)
CAG CGG AAC CGC TCA TTG CCA ATG G
 
β-actin TaqMan probe (SEQ ID NO: 22)
(FAM)ATGCCC-T(TAMRA)-CCCCCATGCCATCCTGCGTp-3'
 
GAPDH forward primer (SEQ ID NO: 23)
GAAGGTGAAGGTCGGAGT
 
GAPDH reverse primer (SEQ ID NO: 24)
GAAGATGGTGATGGGATTTC
 
GAPDH TaqMan probe (SEQ ID NO: 25)
(FAM)CAAGCTTCCCGTTCTCAGCC(TAMRA)-3'
 FAM: 6-carboxy-fluorescein
TAMRA: 6-carboxy-N,N,N',N'-tetramethylrhodamine

As a result of quantitative PCR, the expression levels of 6 genes in the respiratory tract epithelial cells, where the genes were selected in Example 1, were elevated by twofold or more in three different respiratory tract epithelial cells due to IL-4 or IL-13 stimulation. Based on these results, elevation of expression levels of these indicator genes in response to IL-4 and IL-13 could be predicted in respiratory tract epithelial cells.

The indicator genes of this invention show common behavior among different lots of bronchial epithelial cells by IL-4 and IL-13 stimulation known to have close relationship to allergic reactions. Therefore, the indicator genes of this invention can be thought to be important genes regulating the progress of allergic reactions.

### Industrial Applicability

By this invention, genes that increase their expression in respiratory tract epithelial cells stimulated with IL-4 or IL-13 were found. It is highly probable that genes whose expression elevates in respiratory tract epithelial cells stimulated with IL-4 or IL-13 are the fundamental causes of allergic symptoms in bronchial asthma. Therefore, the indicator genes provided by this invention become useful indicators for reliably knowing whether the bronchial asthma has been caused by allergic symptoms. By enabling reliable diagnosis of bronchial asthma caused by allergies, accurate therapy can be selected at an early stage.

IL-4 and IL-13 are important factors for enhancing allergic reactions. Therefore, genes that increase their expression accompanying stimulation by these factors are considered to accomplish important roles in pathological formation of allergic symptoms. Furthermore, any of the indicator genes provided by this invention showed clear enhancement of expression in a plurality of respiratory tract epithelial cells. Studies focusing on the variance in gene expression level accompanying stimulation by IL-4 or IL-13 are not novel. However, the genes provided by this invention are all allergy related genes found by stringent criteria such as those described herein. Therefore, the indicator genes of this invention can be considered to be genes accomplishing an important role in allergic reactions compared to known allergy related genes obtained by similar approaches. The reason is that the indicator genes of this invention always respond sensitively to IL-4 or IL-13 stimulation in different cells. This supports the fact that the existence of indicator genes of this invention is indispensable to allergic reactions.

Thus, since excess expression of any indicator genes of this invention is linked to pathology, not only does suppression of these genes become the target of therapeutic strategy for allergic diseases, but also utility can be expected for these genes as novel clinical diagnostic indicators for monitoring in such novel treatment.

The expression level of indicator genes provided by this invention can be easily known, regardless of the type of allergen. Therefore, the pathology of an allergic reaction can be understood overall.

Additionally, the method of testing for allergies of this invention has low invasiveness towards patients since analysis of expression level can be carried out using a biological sample as the sample. Furthermore, regarding gene expression analysis, highly sensitive measurements are possible using small amounts of samples. Year after year, high throughput methods and decrease in cost are progressing in gene analysis technology. Therefore, in the near future, the method of testing for allergies of this invention is expected to become an important bed-side diagnostic method. In this sense, diagnostic value of these pathology related genes is high.

## Claims

1. A method of testing for an allergic disease, the method comprising the steps of:
a) measuring the expression level of one or more genes selected from the group consisting of carboxypeptidase M, cathepsin C, endothelin-A receptor, osteoblast specific factor 2, DD96, and CYP1B1 in a biological sample from a subject, and
b) comparing the expression level measured in (a) with that in a biological sample from a normal healthy subject.

2. The method of claim 1, wherein the allergic disease is bronchial asthma.

3. The method of claim 1, wherein the expression level is measured by PCR of the cDNA for the one or more genes.

4. The method of claim 1, wherein the expression level is measured by detecting the protein encoded by the one or more genes.

5. A reagent for testing for an allergic disease, said reagent comprising an oligonucleotide that is at least 15 nucleotides long and that has a nucleotide sequence complementary to a polynucleotide having a nucleotide sequence of one or more genes selected from the group consisting of carboxypeptidase M, cathepsin C, endothelin-A receptor, osteoblast specific factor 2, DD96, and CYP1B1 or to a complementary strand of the polynucleotide.

6. A reagent for testing for an allergic disease, said reagent comprising an antibody that recognizes a peptide having an amino acid sequence of one or more proteins selected from the group consisting of carboxypeptidase M, cathepsin C endothelin-Areceptor, osteoblast specific factor 2, DD96, and CYP1B1.

7. A method of screening for a therapeutic agent for an allergic disease, the method comprising the steps of:
(1) contacting a candidate compound with a cell that expresses one or more genes selected from the group consisting of carboxypeptidase M, cathepsin C, endothelin-A receptor, osteoblast specific factor 2, DD96, and CYP1B1, and/or one or more genes functionally equivalent thereto,
(2) measuring the expression level of the one or more genes, and
(3) selecting a compound that lowers the expression level, compared to a control.

8. The method of claim 7, wherein the cell is a respiratory tract epithelial cell line.

9. A method of screening for a therapeutic agent for an allergic disease, the method comprising the steps of:
(1) administering a candidate compound to a test animal,
(2) measuring, in a biological sample from the test animal, the expression level of one or more genes selected from the group consisting of carboxypeptidase M, cathepsin C, endothelin-A receptor, osteoblast specific factor 2, DD96, and CYP1B1, and/or one or more genes functionally equivalent thereto, and
(3) selecting a compound that lowers the expression level of the one or more genes, compared to a control.

10. A method of screening for a therapeutic agent for an allergic disease, the method comprising the steps of:
(1) contacting a candidate substance with a cell transfected with a vector having a transcription regulatory region of one or more genes selected from the group consisting of carboxypeptidase M, cathepsin C, endothelin-A receptor, osteoblast specific factor 2, DD96, and CYP1B1, and/or one or more genes functionally equivalent thereto, and a reporter gene that is expressed under the control of the transcription regulatory region,
(2) measuring activity of the reporter gene, and
(3) selecting a compound that lowers the expression level of the reporter gene, compared to a control.

11. A method of screening for a therapeutic agent for an allergic disease, the method comprising the steps of:
(1) contacting a candidate substance with one or more proteins selected from the group consisting of carboxypeptidase M, cathepsin C, endothelin-A receptor, osteoblast specific factor 2, DD96, and CYP1B1, and/or one or more proteins functionally equivalent thereto,
(2) measuring activity of the one or more proteins, and
(3) selecting a compound that lowers the activity, compared to a control.

12. A therapeutic agent for an allergic disease, said agent comprising, as an active ingredient, a compound obtained by the method of any one of claims 7, 9, 10, and 11.

13. A therapeutic agent for an allergic disease, said agent comprising, as a major component, an antisense DNA against one or more genes selected from the group consisting of carboxypeptidase M, cathepsin C, endothelin-A receptor, osteoblast specific factor 2, DD96, and CYP1B1 or against a portion thereof.

14. A therapeutic agent for an allergic disease, said agent comprising, as a major component, an antibody that binds to one or more proteins selected from the group consisting of carboxypeptidase M, cathepsin C, endothelin-A receptor, osteoblast specific factor 2, DD96, and CYP1B1.

15. Use of a transgenic non-human vertebrate in which the expression level of one or more genes selected from the group consisting of carboxypeptidase M, cathepsin C, endothelin-A receptor, osteoblast specific factor 2, DD96, and CYP1B1, and/or one or more genes functionally equivalent thereto is elevated in respiratory tract epithelial cells, as an animal model for an allergic disease.

16. A kit for screening for a candidate compound for a therapeutic agent for an allergic disease, the kit comprising
a polynucleotide that is at least 15 nucleotides long and that hybridizes to a nucleotide sequence of one or more genes selected from the group consisting of carboxypeptidase M, cathepsin C, endothelin-A receptor, osteoblast specific factor 2, DD96(MAP17), and CYP1B1 or to a complementary sequence thereof, and
a cell that expresses a gene comprising a nucleotide sequence of one or more genes selected from the group consisting of carboxypeptidase M, cathepsin C, endothelin-A receptor, osteoblast specific factor 2, DD96(MAP17), and CYP1B1.

17. A kit for screening for a candidate compound for a therapeutic agent for an allergic disease, the kit comprising
an antibody that recognizes a peptide having an amino acid sequence of one or more proteins selected from the group consisting of carboxypeptidase M, cathepsinC, endothelin-A receptor , osteoblast specific factor 2, DD96(MAP17), and CYP1B1, and
a cell that expresses a gene comprising a nucleotide sequence of one or more genes selected from the group consisting of carboxypeptidase M, cathepsin C, endothelin-A receptor, osteoblast specific factor 2, DD96 (MAP17), and CYP1B1.


SEQUENCE LISTING

<110> Genox Research, Inc.

<120> METHOD OF TESTING FOR ALLERGIC DISEASE

<130> G1-A0008P

<140>
<141>

<150> JP 2000-396166
<151> 2000-12-26

<160> 37

<170> PatentIn Ver. 2.1

<210> 1
<211> 63
<212> DNA
<213> Artificial Sequence

<220>
<213> Description of Artificial Sequence:an artificially
      synthesized T7-d(T)24 primer sequence

<400> 1
ggccagtgaa ttgtaatacg actcactata gggaggcggt tttttttttt tttttttttt 60
ttt 63

<210> 2
<211> 23
<212> DNA
<213> Artificial Sequence

<220>
<223> Description of Artificial Sequence:an artificially
synthesized primer sequence

<400> 2
acagagacgt ttgtcctctc tgc 23

<210> 3
<211> 21
<212> DNA
<213> Artificial Sequence

<220>
<223> Description of Artificial Sequence:an artificially

synthesized primer sequence

<400> 3
atgccccagt tgcttgaaca c                                                21

<210> 4
<211> 27
<212> DNA
<213> Artificial Sequence

<220>
<223> Description of Artificial Sequence:an artificially
      synthesized TaqMan probe sequence

<220>
<221> misc_binding
<222> (1)
<223> Label FAM (6-carboxy-fluorescein)

<220>
<221> misc_binding
<222> (27)
<223> Label TAMRA
      (6-carboxy-N,N,N',N'-tetrametylrhodamine)

<400> 4
ccctcgtggc cagttaccca tttgata                                    27

<210> 5
<211> 22
<212> DNA
<213> Artificial Sequence

<220>
<223> Description of Artificial Sequence:an artificially
      synthesized primer sequence

<400> 5
tctcagaccc caatcctaag cc                                         22

<210> 6
<211> 22
<212> DNA
<213> Artificial Sequence

<220>
<223> Description of Artificial Sequence:an artificially
      synthesized primer sequence

<400> 6

ctgcaataag gtatgggaag cc                                                22

<210> 7

<211> 30

<212> DNA

<213> Artificial Sequence

<220>

<223> Description of Artificial Sequence:an artificially

      synthesized TaqMan probe sequence

<220>

<221> misc_binding

<222> (1)

<223> Label FAM (6-carboxy-fluorescein)

<220>

<221> misc_binding

<222> (30)

<223> Label TAMRA

      (6-carboxy-N,N,N',N'-tetramethylrhodamine)

<400> 7

tcttgtagcc agtatgctca aggctgtgaa                                        30

<210> 8

<211> 23

<212> DNA

<213> Artificial Sequence

<220>

<223> Description of Artificial Sequence:an artificially

synthesized primer sequence

<400> 8

acctctgcgc tcttagtgtt gac 23

<210> 9

<211> 22

<212> DNA

<213> Artificial Sequence

<220>

<223> Description of Artificial Sequence:an artificially

synthesized primer sequence

<400> 9

gcagttacca aaggaatccc aa 22

<210> 10

<211> 30

<212> DNA

<213> Artificial Sequence

<220>

<223> Description of Artificial Sequence:an artificially

synthesized TaqMan probe sequence

<220>

<221> misc_binding

<222> (1)

<223> Label FAM (6-carboxy-fluorescein)

<220>

<221> misc_binding

<222> (30)

<223> Label TAMRA

(6-carboxy-N,N,N',N'-tetramethylrhodamine)

<400> 10

tacagagcag ttgcctcctg gagtcgtgtt 30

<210> 11

<211> 21

<212> DNA

<213> Artificial Sequence

<220>

<223> Description of Artificial Sequence:an artificially

synthesized primer sequence

<400> 11

agcaaaccac cttcacggat c 21

<210> 12

<211> 23

<212> DNA

<213> Artificial Sequence

<220>

<223> Description of Artificial Sequence:an artificially

synthesized primer sequence

<400> 12

ggtgccagca aagtgtattc tcc 23

<210> 13

<211> 28

<212> DNA

<213> Artificial Sequence

<220>

<223> Description of Artificial Sequence:an artificially

synthesized TaqMan probe sequence

<220>

<221> misc_binding

<222> (1)

<223> Label FAM (6-carboxy-fluorescein)

<220>

<221> misc_binding

<222> (28)

<223> Label TAMRA

(6-carboxy-N,N,N',N'-tetramethylrhodamine)

<400> 13

aattaggctt ggcatctgct ctgaggcc 28

<210> 14

<211> 23

<212> DNA
<213> Artificial Sequence

<220>
<223> Description of Artificial Sequence:an artificially
      synthesized primer sequence

<400> 14
gcctttgcag tcaaccactt ctg                                              23

<210> 15
<211> 22
<212> DNA
<213> Artificial Sequence

<220>
<223> Description of Artificial Sequence:an artificially
      synthesized primer sequence

<400> 15
tctgttccca ccaggactcc at                                               22

<210> 16
<211> 26

<212> DNA

<213> Artificial Sequence

<220>

<223> Description of Artificial Sequence:an artificially

synthesized TaqMan probe sequence

<220>

<221> misc_binding

<222> (1)

<223> Label FAM (6-carboxy-fluorescein)

<220>

<221> misc_binding

<222> (26)

<223> Label TAMRA

(6-carboxy-N,N,N',N'-tetramethylrhodamine)

<400> 16

atgatcctga ccgtcggaaa caaggc 26

<210> 17

<211> 20

<212> DNA

<213> Artificial Sequence

<220>

<223> Description of Artificial Sequence:an artificially

synthesized primer sequence

<400> 17

ttatgaagcc atgcgcttct 20

<210> 18

<211> 21

<212> DNA

<213> Artificial Sequence

<220>

<223> Description of Artificial Sequence:an artificially

synthesized primer sequence

<400> 18

aagacagagg tgttggcagt g 21

<210> 19

<211> 29

<212> DNA

<213> Artificial Sequence

<220>

<223> Description of Artificial Sequence:an artificially

synthesized TaqMan probe sequence

<220>

<221> misc_binding

<222> (1)

<223> Label FAM (6-carboxy-fluorescein)

<220>

<221> misc_binding

<222> (29)

<223> Label TAMRA

(6-carboxy-N,N,N',N'-tetramethylrhodamine)

<400> 19

cagctttgtg cctgtcacta ttcctcatg 29

<210> 20

<211> 25

<212> DNA

<213> Artificial Sequence

<220>

<223> Description of Artificial Sequence:an artificially
      synthesized primer sequence

<400> 20
tcacccacac tgtgcccatc tacga                                          25

<210> 21
<211> 25
<212> DNA
<213> Artificial Sequence

<220>
<223> Description of Artificial Sequence:an artificially
      synthesized primer sequence

<400> 21
cagcggaacc gctcattgcc aatgg                                          25

<210> 22
<211> 26
<212> DNA
<213> Artificial Sequence

<220>

<223> Description of Artificial Sequence:an artificially
      synthesized TaqMan probe sequence

<220>
<221> misc_binding
<222> (1)
<223> Label FAM (6-carboxy-fluorescein)

<220>
<221> misc_binding
<222> (7)
<223> Label TAMRA
      (6-carboxy-N,N',N'-tetramethylrhodamine)

<400> 22
atgccctccc ccatgccatc ctgcgt                                          26

<210> 23
<211> 18
<212> DNA
<213> Artificial Sequence

<220>
<223> Description of Artificial Sequence:an artificially
      synthesized primer sequence

<400> 23

gaaggtgaag gtcggagt                                                    18

<210> 24

<211> 20

<212> DNA

<213> Artificial Sequence

<220>

<223> Description of Artificial Sequence:an artificially

      synthesized primer sequence

<400> 24

gaagatggtg atgggatttc                                                  20

<210> 25

<211> 20

<212> DNA

<213> Artificial Sequence

<220>

<223> Description of Artificial Sequence:an artificially

      synthesized TaqMan probe sequence

<220>

<221> misc_binding

<222> (1)

<223> Label FAM (6-carboxy-fluorescein)

<220>

<221> misc_binding

<222> (20)

<223> Label TAMRA

(6-carboxy-N,N,N',N'-tetramethylrhodamine)

<400> 25

caagcttccc gttctcagcc 20

<210> 26

<211> 2085

<212> DNA

<213> Homo sapiens

<400> 26

gcatttcttc cttctgcgta tgggacagga ccctttctgg aatgggggtc ttatgaccta 60

caatcaaaca agaacatgga cttcccgtgc ctctggctag ggctgttgct gcctttggta 120

gctgcgctgg atttcaacta ccaccgccag gaagggatgg aagcgttttt gaagactgtt 180

gcccaaaact acagttctgt cactcactta cacagtattg ggaaatctgt gaaaggtaga 240

aacctgtggg ttcttgttgt ggggcggttt ccaaaggaac acagaattgg gattccagag 300

ttcaaatacg tggcaaatat gcatggagat gagactgttg ggcgggagct gctgctccat 360

ctgattgact atctcgtaac cagtgatggc aaagaccctg aaatcacaaa tctgatcaat 420

agtacccgga tacacatcat gccttccatg aacccagatg gatttgaagc cgtcaaaaag 480

cctgactgtt actacagcat cggaagggaa aattataacc agtatgactt gaatcgaaat 540

ttccccgatg cttttgaata taataatgtc tcaaggcagc ctgaaactgt ggcagtcatg 600

aagtggctga aaacagagac gtttgtcctc tctgcaaacc tccatggtgg tgccctcgtg 660

gccagttacc catttgataa tggtgttcaa gcaactgggg cattatactc ccgaagctta 720

acgcctgatg atgatgtttt tcaatatctt gcacatacct atgcttcaag aaatcccaac 780

atgaagaaag gagacgagtg taaaaacaaa atgaactttc ctaatggtgt tacaaatgga 840

tactcttggt atccactcca aggtggaatg caagattaca actacatctg ggcccagtgt 900

tttgaaatta cgttggagct gtcatgctgt aaatatcctc gtgaggagaa gcttccatcc 960

ttttggaata ataacaaagc ctcattaatt gaatatataa agcaggtgca cctaggtgta 1020

aagggtcaag tttttgatca gaatggaaat ccattaccca atgtaattgt ggaagtccaa 1080

gacagaaaac atatctgccc ctatagaacc aacaaatatg gagagtatta tctccttctc 1140

ttgcctgggt cttatattat aaatgttaca gtccctggac atgatccaca catcacaaag 1200

gtgattattc cggagaaatc ccagaacttc agtgctctta aaaaggatat tctacttcca 1260

ttccaagggc aattggattc tatcccagta tcaaatcctt catgcccaat gattcctcta 1320

tacagaaatt tgccagacca ctcagctgca acaaagccta gtttgttctt atttttagtg 1380

agtcttttgc acatattctt caaataaagt aaaatgtgaa actcaaccca catcaccacc 1440

tggaatcagg gattgctcac tccaggttac tgcaacccta actcactcta gtgggacctt 1500

gactggagaa actccacgat cttcctgaag aagagaaatg gatgtttcca aattccacaa 1560

taagcaatat gtggtgataa tgaaaagaat gattcagtct tgacggtgaa tggaagacac 1620

ttacctaaca agtactgctc atttacactc aaattaatct tgaagtagtc ttaaaatgtg 1680

taagaagtta aaacttgaga agcaaaaaat gcctgcaaaa agaagatcat tttgtataca 1740

gagaaccgga tgaatataag caatgaagat gaacatttat tgatcttcta catacaagac 1800

ttcaccataa ggccaggagc agtgctcacg ccttgtaatc ccagcacttt gggaggccaa 1860

ggtgggcgga tcaccttgag gtcaggagtt caagaccagc ctgaccaaca tggtgaaacc 1920

ctgtctctac taaatattag cggggtgtgg tggcgggcac ctgtagtcgc agcctttcgg 1980

gaggctgaga caggagaatc gcttgaaccc tagaggcgga gtttgcagtg agccgagata 2040

gtgccattgt actccagctt gggcaacaga gtaagactct gtctc 2085

<210> 27

<211> 443

<212> PRT

<213> Homo sapiens

<400> 27

Met Asp Phe Pro Cys Leu Trp Leu Gly Leu Leu Leu Pro Leu Val Ala

1               5                   10                  15

Ala Leu Asp Phe Asn Tyr His Arg Gln Glu Gly Met Glu Ala Phe Leu

            20                  25                  30

Lys Thr Val Ala Gln Asn Tyr Ser Ser Val Thr His Leu His Ser Ile

        35                  40                  45

Gly Lys Ser Val Lys Gly Arg Asn Leu Trp Val Leu Val Val Gly Arg

    50                  55                  60

Phe Pro Lys Glu His Arg Ile Gly Ile Pro Glu Phe Lys Tyr Val Ala

65                  70                  75                  80

Asn Met His Gly Asp Glu Thr Val Gly Arg Glu Leu Leu Leu His Leu

                85                  90                  95

Ile Asp Tyr Leu Val Thr Ser Asp Gly Lys Asp Pro Glu Ile Thr Asn

            100                 105                 110

Leu Ile Asn Ser Thr Arg Ile His Ile Met Pro Ser Met Asn Pro Asp

        115                 120                 125

Gly Phe Glu Ala Val Lys Lys Pro Asp Cys Tyr Tyr Ser Ile Gly Arg

    130                 135                 140

Glu Asn Tyr Asn Gln Tyr Asp Leu Asn Arg Asn Phe Pro Asp Ala Phe

145                 150                 155                 160

Glu Tyr Asn Asn Val Ser Arg Gln Pro Glu Thr Val Ala Val Met Lys

                165                 170                 175

Trp Leu Lys Thr Glu Thr Phe Val Leu Ser Ala Asn Leu His Gly Gly

            180                 185                 190

Ala Leu Val Ala Ser Tyr Pro Phe Asp Asn Gly Val Gln Ala Thr Gly

        195                 200                 205

Ala Leu Tyr Ser Arg Ser Leu Thr Pro Asp Asp Asp Val Phe Gln Tyr
    210                 215                 220
Leu Ala His Thr Tyr Ala Ser Arg Asn Pro Asn Met Lys Lys Gly Asp
225                 230                 235                 240
Glu Cys Lys Asn Lys Met Asn Phe Pro Asn Gly Val Thr Asn Gly Tyr
                245                 250                 255
Ser Trp Tyr Pro Leu Gln Gly Gly Met Gln Asp Tyr Asn Tyr Ile Trp
            260                 265                 270
Ala Gln Cys Phe Glu Ile Thr Leu Glu Leu Ser Cys Cys Lys Tyr Pro
        275                 280                 285
Arg Glu Glu Lys Leu Pro Ser Phe Trp Asn Asn Asn Lys Ala Ser Leu
    290                 295                 300
Ile Glu Tyr Ile Lys Gln Val His Leu Gly Val Lys Gly Gln Val Phe
305                 310                 315                 320
Asp Gln Asn Gly Asn Pro Leu Pro Asn Val Ile Val Glu Val Gln Asp
                325                 330                 335
Arg Lys His Ile Cys Pro Tyr Arg Thr Asn Lys Tyr Gly Glu Tyr Tyr
            340                 345                 350

Leu Leu Leu Leu Pro Gly Ser Tyr Ile Ile Asn Val Thr Val Pro Gly
        355                 360                 365

His Asp Pro His Ile Thr Lys Val Ile Ile Pro Glu Lys Ser Gln Asn
    370                 375                 380

Phe Ser Ala Leu Lys Lys Asp Ile Leu Leu Pro Phe Gln Gly Gln Leu
385                 390                 395                 400

Asp Ser Ile Pro Val Ser Asn Pro Ser Cys Pro Met Ile Pro Leu Tyr
                405                 410                 415

Arg Asn Leu Pro Asp His Ser Ala Ala Thr Lys Pro Ser Leu Phe Leu
            420                 425                 430

Phe Leu Val Ser Leu Leu His Ile Phe Phe Lys
        435                 440

<210> 28
<211> 1838
<212> DNA
<213> Homo sapiens

<400> 28

aattcttcac ctcttttctc agctccctgc agcatgggtg ctgggccctc cttgctgctc 60

gccgccctcc tgctgcttct ctccggcgac ggcgccgtgc gctgcgacac acctgccaac 120

tgcacctatc ttgacctgct gggcacctgg gtcttccagg tgggctccag cggttcccag 180

cgcgatgtca actgctcggt tatgggacca caagaaaaaa aagtagtggt gtaccttcag 240

aagctggata cagcatatga tgaccttggc aattctggcc atttcaccat catttacaac 300

caaggctttg agattgtgtt gaatgactac aagtggtttg ccttttttaa gtataaagaa 360

gagggcagca aggtgaccac ttactgcaac gagacaatga ctgggtgggt gcatgatgtg 420

ttgggccgga actgggcttg tttcaccgga aagaaggtgg gaactgcctc tgagaatgtg 480

tatgtcaaca cagcacacct taagaattct caggaaaagt attctaatag gctctacaag 540

tatgatcaca actttgtgaa agctatcaat gccattcaga agtcttggac tgcaactaca 600

tacatggaat atgagactct taccctggga gatatgatta ggagaagtgg tggccacagt 660

cgaaaaatcc caaggcccaa acctgcacca ctgactgctg aaatacagca aaagattttg 720

catttgccaa catcttggga ctggagaaat gttcatggta tcaattttgt cagtcctgtt 780

cgaaaccaag catcctgtgg cagctgctac tcatttgctt ctatgggtat gctagaagcg 840

agaatccgta tactaaccaa caattctcag accccaatcc taagccctca ggaggttgtg 900

tcttgtagcc agtatgctca aggctgtgaa ggcggcttcc cataccttat tgcaggaaag 960

tacgcccaag attttgggct ggtggaagaa gcttgcttcc cctacacagg cactgattct 1020

ccatgcaaaa tgaaggaaga ctgctttcgt tattactcct ctgagtacca ctatgtagga 1080

ggtttctatg gaggctgcaa tgaagccctg atgaagcttg agttggtcca tcatgggccc 1140

atggcagttg cttttgaagt atatgatgac ttcctccact acaaaaaggg gatctaccac 1200

cacactggtc taagagaccc tttcaacccc tttgagctga ctaatcatgc tgttctgctt 1260

gtgggctatg gcactgactc agcctctggg atggattact ggattgttaa aaacagctgg 1320

ggcaccggct ggggtgagaa tggctacttc cggatccgca gaggaactga tgagtgtgca 1380

attgagagca tagcagtggc agccacacca attcctaaat tgtagggtat gccttccagt 1440

atttcataat gatctgcatc agttgtaaag gggaattggt atattcacag actgtagact 1500

ttcagcagca atctcagaag cttacaaata gatttccatg aagatatttg tcttcagaat 1560

taaaactgcc cttaatttta atataccttt caatcggcca ctggccattt ttttctaagt 1620

attcaattaa gtgggaattt tctggaagat ggtcagctat gaagtaatag agtttgctta 1680

atcatttgta attcaaacat gctatatttt ttaaaatcaa tgtgaaaaca tagacttatt 1740

tttaaattgt accaatcaca agaaaataat ggcaataatt atcaaaactt ttaaaataga 1800

tgctcatatt tttaaaataa agttttaaaa ataactgc 1838

<210> 29

<211> 463

<212> PRT

<213> Homo sapiens

<400> 29

Met Gly Ala Gly Pro Ser Leu Leu Leu Ala Ala Leu Leu Leu Leu Leu

1               5                   10                  15

Ser Gly Asp Gly Ala Val Arg Cys Asp Thr Pro Ala Asn Cys Thr Tyr

            20                  25                  30

Leu Asp Leu Leu Gly Thr Trp Val Phe Gln Val Gly Ser Ser Gly Ser

        35                  40                  45

Gln Arg Asp Val Asn Cys Ser Val Met Gly Pro Gln Glu Lys Lys Val

    50                  55                  60

Val Val Tyr Leu Gln Lys Leu Asp Thr Ala Tyr Asp Asp Leu Gly Asn

65                  70                  75                  80

Ser Gly His Phe Thr Ile Ile Tyr Asn Gln Gly Phe Glu Ile Val Leu

                85                  90                  95

Asn Asp Tyr Lys Trp Phe Ala Phe Phe Lys Tyr Lys Glu Glu Gly Ser

            100                 105                 110

Lys Val Thr Thr Tyr Cys Asn Glu Thr Met Thr Gly Trp Val His Asp

        115                 120                 125

Val Leu Gly Arg Asn Trp Ala Cys Phe Thr Gly Lys Lys Val Gly Thr

    130                 135                 140

Ala Ser Glu Asn Val Tyr Val Asn Thr Ala His Leu Lys Asn Ser Gln

145                 150                 155                 160

Glu Lys Tyr Ser Asn Arg Leu Tyr Lys Tyr Asp His Asn Phe Val Lys

                165                 170                 175

Ala Ile Asn Ala Ile Gln Lys Ser Trp Thr Ala Thr Thr Tyr Met Glu

            180                 185                 190

Tyr Glu Thr Leu Thr Leu Gly Asp Met Ile Arg Arg Ser Gly Gly His

        195                 200                 205

Ser Arg Lys Ile Pro Arg Pro Lys Pro Ala Pro Leu Thr Ala Glu Ile
    210                 215                 220
Gln Gln Lys Ile Leu His Leu Pro Thr Ser Trp Asp Trp Arg Asn Val
225                 230                 235                 240
His Gly Ile Asn Phe Val Ser Pro Val Arg Asn Gln Ala Ser Cys Gly
                245                 250                 255
Ser Cys Tyr Ser Phe Ala Ser Met Gly Met Leu Glu Ala Arg Ile Arg
            260                 265                 270
Ile Leu Thr Asn Asn Ser Gln Thr Pro Ile Leu Ser Pro Gln Glu Val
        275                 280                 285
Val Ser Cys Ser Gln Tyr Ala Gln Gly Cys Glu Gly Gly Phe Pro Tyr
    290                 295                 300
Leu Ile Ala Gly Lys Tyr Ala Gln Asp Phe Gly Leu Val Glu Glu Ala
305                 310                 315                 320
Cys Phe Pro Tyr Thr Gly Thr Asp Ser Pro Cys Lys Met Lys Glu Asp
                325                 330                 335
Cys Phe Arg Tyr Tyr Ser Ser Glu Tyr His Tyr Val Gly Gly Phe Tyr
            340                 345                 350

Gly Gly Cys Asn Glu Ala Leu Met Lys Leu Glu Leu Val His His Gly
        355                 360                 365

Pro Met Ala Val Ala Phe Glu Val Tyr Asp Asp Phe Leu His Tyr Lys
    370                 375                 380

Lys Gly Ile Tyr His His Thr Gly Leu Arg Asp Pro Phe Asn Pro Phe
385                 390                 395                 400

Glu Leu Thr Asn His Ala Val Leu Leu Val Gly Tyr Gly Thr Asp Ser
                405                 410                 415

Ala Ser Gly Met Asp Tyr Trp Ile Val Lys Asn Ser Trp Gly Thr Gly
            420                 425                 430

Trp Gly Glu Asn Gly Tyr Phe Arg Ile Arg Arg Gly Thr Asp Glu Cys
        435                 440                 445

Ala Ile Glu Ser Ile Ala Val Ala Ala Thr Pro Ile Pro Lys Leu
    450                 455                 460

<210> 30
<211> 2595
<212> DNA

<213> Homo sapiens

<400> 30
   gtctgttcct tcccccagtc atgcctctgc tgctgctgtt accagtccaa aagtctgatg 60
   acctcggtcc ccatgaacgg aacaagcatc cagtggaaga accacgatca aaacaaccac 120
   aacacagacc ggagcagcca taaggacagc atgaactgac cacccttaga agcactcctc 180
   ggtactccca taatcctctc ggagaaaaaa atcacaaggc aactgtgact ccgggaatct 240
   cttctctgat ccttcttcct taattcactc ccacacccaa gaagaaatgc tttccaaaac 300
   cgcaaggtag actggtttat ccacccacaa catctacgaa tcgtacttct ttaattgatc 360
   taatttacat attctgcgtg ttgtattcag cactaaaaaa tggtgggagc tgggggagaa 420
   tgaagactgt taaatgaaac cagaaggata tttactactt ttgcatgaaa atagagcttt 480
   caagtacatg gctagctttt atggcagttc tggtgaatgt tcaatgggaa ctggtcacca 540
   tgaaacttta gagattaacg acaagatttt ctactttttt taagtgattt tttgtccttc 600
   agccaaacac aatatgggct caggtcactt ttatttgaaa tgtcatttgg tgccagtatt 660
   ttttaactgc ataatagcct aacatgatta tttgaactta tttacacata gtttgaaaaa 720
   aaaaagacaa aaatagtatt caggtgagca attagattag tattttccac gtcactattt 780
   atttttttaa aacacaaatt ctaaagctac aacaaatact acaggccctt aaagcacagt 840
   ctgatgacac atttggcagt ttaatagatg ttactcaaag aattttttaa gaactgtatt 900
   ttatttttta aatggtgttt tattacaagg gaccttgaac atgttttgta tgttaaattc 960
   aaaagtaatg cttcaatcag atagttcttt ttcacaagtt caatctgttt ttcatgtaaa 1020
   ttttgtatga aaaatcaatg tcaagtacca aaatgttaat gtatgtgtca tttaactctg 1080
   cctgagactt tcagtgcact gtatatagaa gtctaaaaca cacctaagag aaaaagatcg 1140
   aatttttcag atgattcgga aattttcatt caggtatttg taatagtgac atatatatgt 1200
   atatacatat cacctcctat tctcttaatt tttgttaaaa tgttaactgg cagtaagtct 1260
   tttttgatca ttcccttttc catataggaa acataatttt gaagtggcca gatgagttta 1320
   tcatgtcagt gaaaaataat tacccacaaa tgccaccagt aacttaacga ttcttcactt 1380
   cttggggttt tcagtatgaa cctaactccc caccccaaca tctccctccc acattgtcac 1440
   catttcaaag ggcccacagt gacttttgct gggcattttc ccagatgttt acagactgtg 1500
   agtacagcag aaaatctttt actagtgtgt gtgtgtatat atataaacaa ttgtaaattt 1560
   cttttagccc atttttctag actgtctctg tggaatatat ttgtgtgtgt gatatatgca 1620
   tgtgtgtgat ggtatgtatg gatttaatct aatctaataa ttgtgccccg cagttgtgcc 1680
   aaagtgcata gtctgagcta aaatctaggt gattgttcat catgacaacc tgcctcagtc 1740
   cattttaacc tgtagcaacc ttctgcattc ataaatcttg taatcatgtt accattacaa 1800
   atgggatata agaggcagcg tgaaagcaga tgagctgtgg actagcaata tagggttttg 1860
   tttggttggt tggtttgata aagcagtatt tggggtcata ttgtttcctg tgctggagca 1920
   aaagtcatta cactttgaag tattatattg ttcttatcct caattcaatg tggtgatgaa 1980
   attgccaggt tgtctgatat ttctttcaga cttcgccaga cagattgctg ataataaatt 2040
   aggtaagata atttgttggg ccatatttta ggacaggtaa aataacatca ggttccagtt 2100
   gcttgaattg caaggctaag aagtactgcc cttttgtgtg ttagcagtca aatctattat 2160
   tccactggcg catcatatgc agtgatatat gcctataata taagccatag gttcacacca 2220
   ttttgtttag acaattgtct ttttttcaag atgctttgtt tctttcatat gaaaaaaatg 2280
   cattttataa attcagaaag tcatagattt ctgaaggcgt caacgtgcat tttatttatg 2340
   gactggtaag taactgtggt ttactagcag gaatatttcc aatttctacc tttactacat 2400
   cttttcaaca agtaactttg tagaaatgag ccagaagcca aggccctgag ttggcagtgg 2460
   cccataagtg taaaataaaa gtttacagaa accttgcaag tgtctcttca tttttatgta 2520
   gttttccata gaaaatgttt gttacataat gcctgttgca aacctctcct tagtaatatc 2580
   ctggaaagca gttta 2595

<210> 31

<211> 427

<212> PRT

<213> Homo sapiens

<400> 31

Met Glu Thr Leu Cys Leu Arg Ala Ser Phe Trp Leu Ala Leu Val Gly
1               5                   10                  15

Cys Val Ile Ser Asp Asn Pro Glu Arg Tyr Ser Thr Asn Leu Ser Asn
            20                  25                  30

His Val Asp Asp Phe Thr Thr Phe Arg Gly Thr Glu Leu Ser Phe Leu
        35                  40                  45

Val Thr Thr His Gln Pro Thr Asn Leu Val Leu Pro Ser Asn Gly Ser
    50                  55                  60

Met His Asn Tyr Cys Pro Gln Gln Thr Lys Ile Thr Ser Ala Phe Lys
65                  70                  75                  80

Tyr Ile Asn Thr Val Ile Ser Cys Thr Ile Phe Ile Val Gly Met Val
                85                  90                  95

Gly Asn Ala Thr Leu Leu Arg Ile Ile Tyr Gln Asn Lys Cys Met Arg
            100                 105                 110

Asn Gly Pro Asn Ala Leu Ile Ala Ser Leu Ala Leu Gly Asp Leu Ile
        115                 120                 125

Tyr Val Val Ile Asp Leu Pro Ile Asn Val Phe Lys Leu Leu Ala Gly
    130                 135                 140
Arg Trp Pro Phe Asp His Asn Asp Phe Gly Val Phe Leu Cys Lys Leu
145                 150                 155                 160
Phe Pro Phe Leu Gln Lys Ser Ser Val Gly Ile Thr Val Leu Asn Leu
                165                 170                 175
Cys Ala Leu Ser Val Asp Arg Tyr Arg Ala Val Ala Ser Trp Ser Arg
            180                 185                 190
Val Gln Gly Ile Gly Ile Pro Leu Val Thr Ala Ile Glu Ile Val Ser
        195                 200                 205
Ile Trp Ile Leu Ser Phe Ile Leu Ala Ile Pro Glu Ala Ile Gly Phe
    210                 215                 220
Val Met Val Pro Phe Glu Tyr Arg Gly Glu Gln His Lys Thr Cys Met
225                 230                 235                 240
Leu Asn Ala Thr Ser Lys Phe Met Glu Phe Tyr Gln Asp Val Lys Asp
                245                 250                 255
Trp Trp Leu Phe Gly Phe Tyr Phe Cys Met Pro Leu Val Cys Thr Ala

            260                 265                 270

Ile Phe Tyr Thr Leu Met Thr Cys Glu Met Leu Asn Arg Arg Asn Gly

        275                 280                 285

Ser Leu Arg Ile Ala Leu Ser Glu His Leu Lys Gln Arg Arg Glu Val

    290                 295                 300

Ala Lys Thr Val Phe Cys Leu Val Val Ile Phe Ala Leu Cys Trp Phe

305                 310                 315                 320

Pro Leu His Leu Ser Arg Ile Leu Lys Lys Thr Val Tyr Asn Glu Met

                325                 330                 335

Asp Lys Asn Arg Cys Glu Leu Leu Ser Phe Leu Leu Leu Met Asp Tyr

            340                 345                 350

Ile Gly Ile Asn Leu Ala Thr Met Asn Ser Cys Ile Asn Pro Ile Ala

        355                 360                 365

Leu Tyr Phe Val Ser Lys Lys Phe Lys Asn Cys Phe Gln Ser Cys Leu

    370                 375                 380

Cys Cys Cys Cys Tyr Gln Ser Lys Ser Leu Met Thr Ser Val Pro Met

385                 390                 395                 400

Asn Gly Thr Ser Ile Gln Trp Lys Asn His Asp Gln Asn Asn His Asn

                405                 410                 415

Thr Asp Arg Ser Ser His Lys Asp Ser Met Asn

            420                 425

<210> 32

<211> 3213

<212> DNA

<213> Homo sapiens

<400> 32

agagactcaa gatgattccc tttttaccca tgttttctct actattgctg cttattgtta 60

accctataaa cgccaacaat cattatgaca agatcttggc tcatagtcgt atcaggggtc 120

gggaccaagg cccaaatgtc tgtgcccttc aacagatttt gggcaccaaa aagaaatact 180

tcagcacttg taagaactgg tataaaaagt ccatctgtgg acagaaaacg actgttttat 240

atgaatgttg ccctggttat atgagaatgg aaggaatgaa aggctgccca gcagttttgc 300

ccattgacca tgtttatggc actctgggca tcgtgggagc caccacaacg cagcgctatt 360

ctgacgcctc aaaactgagg gaggagatcg agggaaaggg atccttcact tactttgcac 420

cgagtaatga ggcttgggac aacttggatt ctgatatccg tagaggtttg gagagcaacg 480

tgaatgttga attactgaat gctttacata gtcacatgat taataagaga atgttgacca 540

aggacttaaa aaatggcatg attattcctt caatgtataa caatttgggg cttttcatta 600

accattatcc taatggggtt gtcactgtta attgtgctcg aatcatccat gggaaccaga 660

ttgcaacaaa tggtgttgtc catgtcattg accgtgtgct tacacaaatt ggtacctcaa 720

ttcaagactt cattgaagca gaagatgacc tttcatcttt tagagcagct gccatcacat 780

cggacatatt ggaggccctt ggaagagacg gtcacttcac actctttgct cccaccaatg 840
aggcttttga gaaacttcca cgaggtgtcc tagaaaggtt catgggagac aaagtggctt 900
ccgaagctct tatgaagtac cacatcttaa atactctcca gtgttctgag tctattatgg 960
gaggagcagt ctttgagacg ctggaaggaa atacaattga gataggatgt gacggtgaca 1020
gtataacagt aaatggaatc aaaatggtga acaaaaagga tattgtgaca aataatggtg 1080
tgatccattt gattgatcag gtcctaattc ctgattctgc caaacaagtt attgagctgg 1140
ctggaaaaca gcaaaccacc ttcacggatc ttgtggccca attaggcttg gcatctgctc 1200
tgaggccaga tggagaatac actttgctgg cacctgtgaa taatgcattt tctgatgata 1260
ctctcagcat ggttcagcgc ctccttaaat taattctgca gaatcacata ttgaaagtaa 1320
aagttggcct taatgagctt tacaacgggc aaatactgga aaccatcgga ggcaaacagc 1380
tcagagtctt cgtatatcgt acagctgtct gcattgaaaa ttcatgcatg gagaaaggga 1440
gtaagcaagg gagaaacggt gcgattcaca tattccgcga gatcatcaag ccagcagaga 1500
aatccctcca tgaaaagtta aaacaagata agcgctttag caccttcctc agcctacttg 1560
aagctgcaga cttgaaagag ctcctgacac aacctggaga ctggacatta tttgtgccaa 1620
ccaatgatgc ttttaaggga atgactagtg aagaaaaaga aattctgata cgggacaaaa 1680
atgctcttca aaacatcatt ctttatcacc tgacaccagg agttttcatt ggaaaaggat 1740
ttgaacctgg tgttactaac attttaaaga ccacacaagg aagcaaaatc tttctgaaag 1800
aagtaaatga tacacttctg gtgaatgaat tgaaatcaaa agaatctgac atcatgacaa 1860
caaatggtgt aattcatgtt gtagataaac tcctctatcc agcagacaca cctgttggaa 1920
atgatcaact gctggaaata cttaataaat taatcaaata catccaaatt aagtttgttc 1980
gtggtagcac cttcaaagaa atccccgtga ctgtctatac aactaaaatt ataaccaaag 2040
ttgtggaacc aaaaattaaa gtgattgaag gcagtcttca gcctattatc aaaactgaag 2100
gacccacact aacaaaagtc aaaattgaag gtgaacctga attcagactg attaaagaag 2160
gtgaaacaat aactgaagtg atccatggag agccaattat taaaaaatac accaaaatca 2220
ttgatggagt gcctgtggaa ataactgaaa aagagacacg agaagaacga atcattacag 2280
gtcctgaaat aaaatacact aggatttcta ctggaggtgg agaaacagaa gaaactctga 2340

agaaattgtt acaagaagag gtcaccaagg tcaccaaatt cattgaaggt ggtgatggtc 2400

atttatttga agatgaagaa attaaaagac tgcttcaggg agacacaccc gtgaggaagt 2460

tgcaagccaa caaaaaagtt caaggttcta gaagacgatt aagggaaggt cgttctcagt 2520

gaaaatccaa aaaccagaaa aaaatgttta tacaacccta agtcaataac ctgaccttag 2580

aaaattgtga gagccaagtt gacttcagga actgaaacat cagcacaaag aagcaatcat 2640

caaataattc tgaacacaaa tttaatattt ttttttctga atgagaaaca tgagggaaat 2700

tgtggagtta gcctcctgtg gtaaaggaat tgaagaaaat ataacacctt acaccctttt 2760

tcatcttgac attaaaagtt ctggctaact ttggaatcca ttagagaaaa atccttgtca 2820

ccagattcat tacaattcaa atcgaagagt tgtgaactgt tatcccattg aaaagaccga 2880

gccttgtatg tatgttatgg atacataaaa tgcacgcaag ccattatctc tccatgggaa 2940

gctaagttat aaaaataggt gcttggtgta caaaactttt tatatcaaaa ggctttgcac 3000

atttctatat gagtgggttt actggtaaat tatgttattt tttacaacta attttgtact 3060

ctcagaatgt ttgtcatatg cttcttgcaa tgcatatttt ttaatctcaa acgtttcaat 3120

aaaaccattt ttcagatata aagagaatta cttcaaattg agtaattcag aaaaactcaa 3180

gatttaagtt aaaaagtggt ttggacttgg gaa 3213

<210> 33

<211> 836

<212> PRT

<213> Homo sapiens

<400> 33

Met Ile Pro Phe Leu Pro Met Phe Ser Leu Leu Leu Leu Leu Ile Val

1               5                   10                  15

Asn Pro Ile Asn Ala Asn Asn His Tyr Asp Lys Ile Leu Ala His Ser
            20                  25                  30
Arg Ile Arg Gly Arg Asp Gln Gly Pro Asn Val Cys Ala Leu Gln Gln
        35                  40                  45
Ile Leu Gly Thr Lys Lys Lys Tyr Phe Ser Thr Cys Lys Asn Trp Tyr
    50                  55                  60
Lys Lys Ser Ile Cys Gly Gln Lys Thr Thr Val Leu Tyr Glu Cys Cys
65                  70                  75                  80
Pro Gly Tyr Met Arg Met Glu Gly Met Lys Gly Cys Pro Ala Val Leu
                85                  90                  95
Pro Ile Asp His Val Tyr Gly Thr Leu Gly Ile Val Gly Ala Thr Thr
            100                 105                 110
Thr Gln Arg Tyr Ser Asp Ala Ser Lys Leu Arg Glu Glu Ile Glu Gly
        115                 120                 125
Lys Gly Ser Phe Thr Tyr Phe Ala Pro Ser Asn Glu Ala Trp Asp Asn
    130                 135                 140
Leu Asp Ser Asp Ile Arg Arg Gly Leu Glu Ser Asn Val Asn Val Glu
145                 150                 155                 160

Leu Leu Asn Ala Leu His Ser His Met Ile Asn Lys Arg Met Leu Thr
                165                 170                 175
Lys Asp Leu Lys Asn Gly Met Ile Ile Pro Ser Met Tyr Asn Asn Leu
            180                 185                 190
Gly Leu Phe Ile Asn His Tyr Pro Asn Gly Val Val Thr Val Asn Cys
        195                 200                 205
Ala Arg Ile Ile His Gly Asn Gln Ile Ala Thr Asn Gly Val Val His
    210                 215                 220
Val Ile Asp Arg Val Leu Thr Gln Ile Gly Thr Ser Ile Gln Asp Phe
225                 230                 235                 240
Ile Glu Ala Glu Asp Asp Leu Ser Ser Phe Arg Ala Ala Ala Ile Thr
                245                 250                 255
Ser Asp Ile Leu Glu Ala Leu Gly Arg Asp Gly His Phe Thr Leu Phe
            260                 265                 270
Ala Pro Thr Asn Glu Ala Phe Glu Lys Leu Pro Arg Gly Val Leu Glu
        275                 280                 285
Arg Phe Met Gly Asp Lys Val Ala Ser Glu Ala Leu Met Lys Tyr His

    290                 295                 300

Ile Leu Asn Thr Leu Gln Cys Ser Glu Ser Ile Met Gly Gly Ala Val
305                 310                 315                 320

Phe Glu Thr Leu Glu Gly Asn Thr Ile Glu Ile Gly Cys Asp Gly Asp
                325                 330                 335

Ser Ile Thr Val Asn Gly Ile Lys Met Val Asn Lys Lys Asp Ile Val
            340                 345                 350

Thr Asn Asn Gly Val Ile His Leu Ile Asp Gln Val Leu Ile Pro Asp
        355                 360                 365

Ser Ala Lys Gln Val Ile Glu Leu Ala Gly Lys Gln Gln Thr Thr Phe
    370                 375                 380

Thr Asp Leu Val Ala Gln Leu Gly Leu Ala Ser Ala Leu Arg Pro Asp
385                 390                 395                 400

Gly Glu Tyr Thr Leu Leu Ala Pro Val Asn Asn Ala Phe Ser Asp Asp
                405                 410                 415

Thr Leu Ser Met Val Gln Arg Leu Leu Lys Leu Ile Leu Gln Asn His
            420                 425                 430

Ile Leu Lys Val Lys Val Gly Leu Asn Glu Leu Tyr Asn Gly Gln Ile
        435                 440                 445

Leu Glu Thr Ile Gly Gly Lys Gln Leu Arg Val Phe Val Tyr Arg Thr
    450                 455                 460

Ala Val Cys Ile Glu Asn Ser Cys Met Glu Lys Gly Ser Lys Gln Gly
465                 470                 475                 480

Arg Asn Gly Ala Ile His Ile Phe Arg Glu Ile Ile Lys Pro Ala Glu
                485                 490                 495

Lys Ser Leu His Glu Lys Leu Lys Gln Asp Lys Arg Phe Ser Thr Phe
            500                 505                 510

Leu Ser Leu Leu Glu Ala Ala Asp Leu Lys Glu Leu Leu Thr Gln Pro
        515                 520                 525

Gly Asp Trp Thr Leu Phe Val Pro Thr Asn Asp Ala Phe Lys Gly Met
    530                 535                 540

Thr Ser Glu Glu Lys Glu Ile Leu Ile Arg Asp Lys Asn Ala Leu Gln
545                 550                 555                 560

Asn Ile Ile Leu Tyr His Leu Thr Pro Gly Val Phe Ile Gly Lys Gly
                565                 570                 575

Phe Glu Pro Gly Val Thr Asn Ile Leu Lys Thr Thr Gln Gly Ser Lys
            580                 585                 590

Ile Phe Leu Lys Glu Val Asn Asp Thr Leu Leu Val Asn Glu Leu Lys
        595                 600                 605

Ser Lys Glu Ser Asp Ile Met Thr Thr Asn Gly Val Ile His Val Val
    610                 615                 620

Asp Lys Leu Leu Tyr Pro Ala Asp Thr Pro Val Gly Asn Asp Gln Leu
625                 630                 635                 640

Leu Glu Ile Leu Asn Lys Leu Ile Lys Tyr Ile Gln Ile Lys Phe Val
                645                 650                 655

Arg Gly Ser Thr Phe Lys Glu Ile Pro Val Thr Val Tyr Thr Thr Lys
            660                 665                 670

Ile Ile Thr Lys Val Val Glu Pro Lys Ile Lys Val Ile Glu Gly Ser
        675                 680                 685

Leu Gln Pro Ile Ile Lys Thr Glu Gly Pro Thr Leu Thr Lys Val Lys
    690                 695                 700

Ile Glu Gly Glu Pro Glu Phe Arg Leu Ile Lys Glu Gly Glu Thr Ile

705                 710                 715                 720

Thr Glu Val Ile His Gly Glu Pro Ile Ile Lys Lys Tyr Thr Lys Ile

                725                 730                 735

Ile Asp Gly Val Pro Val Glu Ile Thr Glu Lys Glu Thr Arg Glu Glu

            740                 745                 750

Arg Ile Ile Thr Gly Pro Glu Ile Lys Tyr Thr Arg Ile Ser Thr Gly

        755                 760                 765

Gly Gly Glu Thr Glu Glu Thr Leu Lys Lys Leu Leu Gln Glu Glu Val

    770                 775                 780

Thr Lys Val Thr Lys Phe Ile Glu Gly Gly Asp Gly His Leu Phe Glu

785                 790                 795                 800

Asp Glu Glu Ile Lys Arg Leu Leu Gln Gly Asp Thr Pro Val Arg Lys

                805                 810                 815

Leu Gln Ala Asn Lys Lys Val Gln Gly Ser Arg Arg Arg Leu Arg Glu

            820                 825                 830

Gly Arg Ser Gln

        835

<210> 34

<211> 927

<212> DNA

<213> Homo sapiens

<400> 34

ggaagtttag gttaactgtc ttaaatttcc aaagctgtaa tcattatttt cattctcaaa 60

gtgatggcct tgtgttttgc tcctctcctc cagggccaga ctgagcccag gttgatttca 120

ggcggacacc aatagactcc acagcagctc caggagccca gacaccggcg gccagaagca 180

aggctaggag ctgctgcagc catgtcggcc ctcagcctcc tcattctggg cctgctcacg 240

gcagtgccac ctgccagctg tcagcaaggc ctggggaacc ttcagccctg gatgcagggc 300

cttatcgcgg tggccgtgtt cctggtcctc gttgcaatcg cctttgcagt caaccacttc 360

tggtgccagg aggagccgga gcctgcacac atgatcctga ccgtcggaaa caaggcagat 420

ggagtcctgg tgggaacaga tggaaggtac tcttcgatgg cggccagttt caggtccagt 480

gagcatgaga atgcctatga gaatgtgccc gaggaggaag gcaaggtccg cagcaccccg 540

atgtaacctt ctctgtggct ccaaccccaa gactcccagg cacatgggat ggatgtccag 600

tgctaccacc caagccccct ccttctttgt gtggaatctg caatagtggg ctgactccct 660

ccagccccat gccggcccta cccgcccttg aagtatagcc agccaaggtt ggagctcaga 720

ccgtgtctag gttggggctc ggctgtggcc ctggggtctc ctgctcagct cagaagagcc 780

ttctggagag gacagtcagc tgagcacctc ccatcctgct cacacgtcct tccccataac 840

tatggaaatg gccctaattt ctgtgaaata aagacttttt gtatttctgg ggctgaggct 900

cagcaacagc ccctcaggct tccaaaa 927

<210> 35

<211> 114

<212> PRT

<213> Homo sapiens

<400> 35

Met Ser Ala Leu Ser Leu Leu Ile Leu Gly Leu Leu Thr Ala Val Pro
1               5                   10                  15

Pro Ala Ser Cys Gln Gln Gly Leu Gly Asn Leu Gln Pro Trp Met Gln
            20                  25                  30

Gly Leu Ile Ala Val Ala Val Phe Leu Val Leu Val Ala Ile Ala Phe
        35                  40                  45

Ala Val Asn His Phe Trp Cys Gln Glu Glu Pro Glu Pro Ala His Met
    50                  55                  60

Ile Leu Thr Val Gly Asn Lys Ala Asp Gly Val Leu Val Gly Thr Asp
65                  70                  75                  80

Gly Arg Tyr Ser Ser Met Ala Ala Ser Phe Arg Ser Ser Glu His Glu
                85                  90                  95

Asn Ala Tyr Glu Asn Val Pro Glu Glu Glu Gly Lys Val Arg Ser Thr
            100                 105                 110

Pro Met

<210> 36

<211> 5102

<212> DNA

<213> Homo sapiens

<400> 36

gcttctgcga ctccagttgt gagagccgca agggcatggg aattgacgcc actcaccgac 60

ccccagtctc aatctcaacg ctgtgaggaa acctcgactt tgccaggtcc ccaagggcag 120

cggggctcgg cgagcgaggc acccttctcc gtccccatcc caatccaagc gctcctggca 180

ctgacgacgc caagagactc gagtgggagt taaagcttcc agtgagggca gcaggtgtcc 240

aggccgggcc tgcgggttcc tgttgacgtc ttgccctagg caaaggtccc agttccttct 300

cggagccggc tgtcccgcgc cactggaaac cgcacctccc cgcagcatgg gcaccagcct 360

cagcccgaac gacccttggc cgctaaaccc gctgtccatc cagcagacca cgctcctgct 420

actcctgtcg gtgctggcca ctgtgcatgt gggccagcgg ctgctgaggc aacggaggcg 480

gcagctccgg tccgcgcccc cgggcccgtt tgcgtggcca ctgatcggaa acgcggcggc 540

ggtgggccag gcggctcacc tctcgttcgc tcgcctggcg cggcgctacg gcgacgtttt 600

ccagatccgc ctgggcagct gccccatagt ggtgctgaat ggcgagcgcg ccatccacca 660

ggccctggtg cagcagggct cggccttcgc cgaccggccg gccttcgcct ccttccgtgt 720

ggtgtccggc ggccgcagca tggctttcgg ccactactcg gagcactgga aggtgcagcg 780

gcgcgcagcc cacagcatga tgcgcaactt cttcacgcgc cagccgcgca gccgccaagt 840

cctcgagggc cacgtgctga gcgaggcgcg cgagctggtg gcgctgctgg tgcgcggcag 900

cgcggacggc gccttcctcg acccgaggcc gctgaccgtc gtggccgtgg ccaacgtcat 960

gagtgccgtg tgtttcggct gccgctacag ccacgacgac cccgagttcc gtgagctgct 1020
cagccacaac gaagagttcg ggcgcacggt gggcgcgggc agcctggtgg acgtgatgcc 1080
ctggctgcag tacttcccca acccggtgcg caccgttttc cgcgaattcg agcagctcaa 1140
ccgcaacttc agcaacttca tcctggacaa gttcttgagg cactgcgaaa gccttcggcc 1200
cggggccgcc ccccgcgaca tgatggacgc ctttatcctc tctgcggaaa agaaggcggc 1260
cggggactcg cacggtggtg gcgcgcggct ggatttggag aacgtaccgg ccactatcac 1320
tgacatcttc ggcgccagcc aggacaccct gtccaccgcg ctgcagtggc tgctcctcct 1380
cttcaccagg tatcctgatg tgcagactcg agtgcaggca gaattggatc aggtcgtggg 1440
gagggaccgt ctgccttgta tgggtgacca gcccaacctg ccctatgtcc tggccttcct 1500
ttatgaagcc atgcgcttct ccagctttgt gcctgtcact attcctcatg ccaccactgc 1560
caacacctct gtcttgggct accacattcc caaggacact gtggtttttg tcaaccagtg 1620
gtctgtgaat catgacccag tgaagtggcc taacccggag aactttgatc cagctcgatt 1680
cttggacaag gatggcctca tcaacaagga cctgaccagc agagtgatga ttttttcagt 1740
gggcaaaagg cggtgcattg gcgaagaact ttctaagatg cagctttttc tcttcatctc 1800
catcctggct caccagtgcg atttcagggc caacccaaat gagcctgcga aaatgaattt 1860
cagttatggt ctaaccatta aacccaagtc atttaaagtc aatgtcactc tcagagagtc 1920
catggagctc cttgatagtg ctgtccaaaa tttacaagcc aaggaaactt gccaataaga 1980
agcaagaggc aagctgaaat tttagaaata ttcacatctt cggagatgag gagtaaaatt 2040
cagttttttt ccagttcctc ttttgtgctg cttctcaatt agcgtttaag gtgagcataa 2100
atcaactgtc catcaggtga ggtgtgctcc atacccagcg gttcttcatg agtagtgggc 2160
tatgcaggag cttctgggag atttttttga gtcaaagact taaagggccc aatgaattat 2220
tatatacata ctgcatcttg gttatttctg aaggtagcat tctttggagt taaaatgcac 2280
atatagacac atacacccaa acacttacac caaactactg aatgaagaag tattttggta 2340
accaggccat ttttggtggg aatccaagat tggtctccca tatgcagaaa tagacaaaaa 2400
gtatattaaa caaagtttca gagtatattg ttgaagagac agagacaagt aatttcagtg 2460
taaagtgtgt gattgaaggt gataagggaa aagataaaga ccagaaattc ccttttcacc 2520

ttttcaggaa aataacttag actctagtat ttatgggtgg atttatcctt ttgccttctg 2580
gtatacttcc ttacttttaa ggataaatca taaagtcagt tgctcaaaaa gaaatcaata 2640
gttgaattag tgagtatagt ggggttccat gagttatcat gaattttaaa gtatgcatta 2700
ttaaattgta aaactccaag gtgatgttgt acctcttttg cttgccaaag tacagaattt 2760
gaattatcag caaagaaaaa aaaaaaagcc agccaagctt taaattatgt gaccataatg 2820
tactgatttc agtaagtctc ataggttaaa aaaaaaagtc accaaatagt gtgaaatata 2880
ttacttaact gtccgtaagc agtatattag tattatcttg ttcaggaaaa ggttgaataa 2940
tatatgcctt gtgtaatatt gaaaattgaa aagtacaact aacgcaacca agtgtgctaa 3000
aaatgagctt gattaaatca accacctatt tttgacatgg aaatgaagca gggtttcttt 3060
tcttcactca aattttggcg aatctcaaaa ttagatccta agatgtgttc ttatttttat 3120
aacatcttta ttgaaattct atttataata cagaatcttg ttttgaaaat aacctaatta 3180
atatattaaa attccaaatt catggcatgc ttaaatttta actaaatttt aaagccattc 3240
tgattattga gttccagttg aagttagtgg aaatctgaac attctcctgt ggaaggcaga 3300
gaaatctaag ctgtgtctgc ccaatgaata atggaaaatg ccatgaatta cctggatgtt 3360
ctttttacga ggtgacaaga gttggggaca gaactcccat tacaactgac caagtttctc 3420
ttctagatga ttttttgaaa gttaacatta atgcctgctt tttggaaagt cagaatcaga 3480
agatagtctt ggaagctgtt tggaaaagac agtggagatg aggtcagttg tgttttttaa 3540
gatggcaatt actttggtag ctgggaaagc ataaagctca aatgaaatgt atgcattcac 3600
atttagaaaa gtgaattgaa gtttcaagtt ttaaagttca ttgcaattaa acttccaaag 3660
aaagttctac agtgtcctaa gtgctaagtg cttattacat tttattaagc tttttggaat 3720
ctttgtacca aaattttaaa aaagggagtt tttgatagtt gtgtgtatgt gtgtgtgggg 3780
tggggggatg gtaagagaaa agagagaaac actgaaaaga aggaaagatg gttaaacatt 3840
ttcccactca ttctgaatta attaatttgg agcacaaaat tcaaagcatg gacatttaga 3900
agaaagatgt ttggcgtagc agagttaaat ctcaaatagg ctattaaaaa agtctacaac 3960
atagcagatc tgttttgtgg tttggaatat taaaaaactt catgtaattt tattttaaaa 4020
tttcatagct gtacttcttg aatataaaaa atcatgccag tatttttaaa ggcattagag 4080

tcaactacac aaagcaggct tgcccagtac atttaaattt tttggcactt gccattccaa 4140

aatattatgc cccaccaagg ctgagacagt gaatttgggc tgctgtagcc tattttttta 4200

gattgagaaa tgtgtagctg caaaaataat catgaaccaa tctggatgcc tcattatgtc 4260

aaccaggtcc agatgtgcta taatctgttt ttacgtatgt aggcccagtc gtcatcagat 4320

gcttgcggca aaagaaagct gtgtttatat ggaagaaagt aaggtgcttg gagtttacct 4380

ggcttattta atatgcttat aacctagtta aagaaaggaa aagaaaacaa aaaacgaatg 4440

aaaataactg aatttggagg ctggagtaat cagattactg ctttaatcag aaaccctcat 4500

tgtgtttcta ccggagagag aatgtatttg ctgacaacca ttaaagtcag aagttttact 4560

ccaggttatt gcaataaagt ataatgttta ttaaatgctt catttgtatg tcaaagcttt 4620

gactctataa gcaaattgct tttttccaaa acaaaaagat gtctcaggtt tgttttgtga 4680

attttctaaa agctttcatg tcccagaact tagcctttac ctgtgaagtg ttactacagc 4740

cttaatattt tcctagtaga tctatattag atcaaatagt tgcatagcag tatatgttaa 4800

tttgtgtgtt tttagctgtg acacaactgt gtgattaaaa ggtatacttt agtagacatt 4860

tataactcaa ggataccttc ttatttaatc ttttcttatt tttgtacttt atcatgaatg 4920

cttttagtgt gtgcataata gctacagtgc atagttgtag acaaagtaca ttctggggaa 4980

acaacattta tatgtagcct ttactgtttg atataccaaa ttaaaaaaaa attgtatctc 5040

attacttata ctgggacacc attaccaaaa taataaaaat cactttcata atcttgaaaa 5100

aa 5102

<210> 37

<211> 543

<212> PRT

<213> Homo sapiens

<400> 37

Met Gly Thr Ser Leu Ser Pro Asn Asp Pro Trp Pro Leu Asn Pro Leu
1               5                   10                  15

Ser Ile Gln Gln Thr Thr Leu Leu Leu Leu Leu Ser Val Leu Ala Thr
            20                  25                  30

Val His Val Gly Gln Arg Leu Leu Arg Gln Arg Arg Arg Gln Leu Arg
        35                  40                  45

Ser Ala Pro Pro Gly Pro Phe Ala Trp Pro Leu Ile Gly Asn Ala Ala
    50                  55                  60

Ala Val Gly Gln Ala Ala His Leu Ser Phe Ala Arg Leu Ala Arg Arg
65                  70                  75                  80

Tyr Gly Asp Val Phe Gln Ile Arg Leu Gly Ser Cys Pro Ile Val Val
                85                  90                  95

Leu Asn Gly Glu Arg Ala Ile His Gln Ala Leu Val Gln Gln Gly Ser
            100                 105                 110

Ala Phe Ala Asp Arg Pro Ala Phe Ala Ser Phe Arg Val Val Ser Gly
        115                 120                 125

Gly Arg Ser Met Ala Phe Gly His Tyr Ser Glu His Trp Lys Val Gln
    130                 135                 140

Arg Arg Ala Ala His Ser Met Met Arg Asn Phe Phe Thr Arg Gln Pro
145                 150                 155                 160

Arg Ser Arg Gln Val Leu Glu Gly His Val Leu Ser Glu Ala Arg Glu
                165                 170                 175

Leu Val Ala Leu Leu Val Arg Gly Ser Ala Asp Gly Ala Phe Leu Asp
            180                 185                 190

Pro Arg Pro Leu Thr Val Val Ala Val Ala Asn Val Met Ser Ala Val
        195                 200                 205

Cys Phe Gly Cys Arg Tyr Ser His Asp Asp Pro Glu Phe Arg Glu Leu
    210                 215                 220

Leu Ser His Asn Glu Glu Phe Gly Arg Thr Val Gly Ala Gly Ser Leu
225                 230                 235                 240

Val Asp Val Met Pro Trp Leu Gln Tyr Phe Pro Asn Pro Val Arg Thr
                245                 250                 255

Val Phe Arg Glu Phe Glu Gln Leu Asn Arg Asn Phe Ser Asn Phe Ile
            260                 265                 270

Leu Asp Lys Phe Leu Arg His Cys Glu Ser Leu Arg Pro Gly Ala Ala

        275                 280                 285

Pro Arg Asp Met Met Asp Ala Phe Ile Leu Ser Ala Glu Lys Lys Ala
    290                 295                 300

Ala Gly Asp Ser His Gly Gly Gly Ala Arg Leu Asp Leu Glu Asn Val
305                 310                 315                 320

Pro Ala Thr Ile Thr Asp Ile Phe Gly Ala Ser Gln Asp Thr Leu Ser
                325                 330                 335

Thr Ala Leu Gln Trp Leu Leu Leu Leu Phe Thr Arg Tyr Pro Asp Val
            340                 345                 350

Gln Thr Arg Val Gln Ala Glu Leu Asp Gln Val Val Gly Arg Asp Arg
        355                 360                 365

Leu Pro Cys Met Gly Asp Gln Pro Asn Leu Pro Tyr Val Leu Ala Phe
    370                 375                 380

Leu Tyr Glu Ala Met Arg Phe Ser Ser Phe Val Pro Val Thr Ile Pro
385                 390                 395                 400

His Ala Thr Thr Ala Asn Thr Ser Val Leu Gly Tyr His Ile Pro Lys
                405                 410                 415

Asp Thr Val Val Phe Val Asn Gln Trp Ser Val Asn His Asp Pro Val
            420                 425                 430
Lys Trp Pro Asn Pro Glu Asn Phe Asp Pro Ala Arg Phe Leu Asp Lys
        435                 440                 445
Asp Gly Leu Ile Asn Lys Asp Leu Thr Ser Arg Val Met Ile Phe Ser
    450                 455                 460
Val Gly Lys Arg Arg Cys Ile Gly Glu Glu Leu Ser Lys Met Gln Leu
465                 470                 475                 480
Phe Leu Phe Ile Ser Ile Leu Ala His Gln Cys Asp Phe Arg Ala Asn
                485                 490                 495
Pro Asn Glu Pro Ala Lys Met Asn Phe Ser Tyr Gly Leu Thr Ile Lys
            500                 505                 510
Pro Lys Ser Phe Lys Val Asn Val Thr Leu Arg Glu Ser Met Glu Leu
        515                 520                 525
Leu Asp Ser Ala Val Gln Asn Leu Gln Ala Lys Glu Thr Cys Gln
    530                 535                 540